# EUROPÄISCHE PATENTSCHRIFT

(11) **EP 0 684 289 B1**
(45) Veröffentlichungstag und Bekanntmachung des Hinweises auf die Patenterteilung: **10.10.2001**
(21) Anmeldenummer: 95107326.1
(22) Anmeldetag: 15.05.1995
(51) Int. Cl.: C09B 57/04, C07D 209/44, C08K 5/3417, C09B 67/22, D06P 1/16

(54) **Thiazol-isoindolenin-Farbstoffe**
Thiazol isoindolenine dyes
Colorants thiazolisoindoléniniques

(30) Priorität: 25.05.1994 DE 4418148; 31.01.1995 DE 19502943
(43) Veröffentlichungstag der Anmeldung: 29.11.1995
(73) Patentinhaber: DyStar Textilfarben GmbH & Co. Deutschland KG, 60318 Frankfurt am Main (DE)
(72) Erfinder: Lorenz, Manfred, Dr., D-51061 Köln (DE); Wanken, Klaus Winfried, D-51373 Leverkusen (DE)

(56) Entgegenhaltungen:
- EP-A- 0 061 094
- EP-A- 0 062 614
- EP-A- 0 132 818
- EP-A- 0 358 148
- EP-A- 0 510 436
- DE-C- 955 178
- FR-A- 1 537 299
- FR-A- 2 307 008
- US-A- 3 499 908

## Beschreibung

Die vorliegende Erfindung betrifft neue Thiazolisoindolenin-Farbstoffe, deren Mischungen, Verfahren zu ihrer Herstellung sowie ihre Verwendung zum Färben von hydrophoben synthetischen Materialien.

Isoindolenin-Derivate sind bereits in EP-A 61 094 beschrieben und in der Elektrofotografie eingesetzt worden. Aus DE-A-2 .615 394 sind Isoindolin-Derivate zum Färben thermoplastischer Kunststoffe in der Schmelze bekannt und in EP-A-358 148 werden feste Isoindolinon- und Isoindolin-Verbindungen zum Färben von Kunststoffen verwendet. Die aus DE-A-1 670 748 bekannten Farbstoffe der Isoindolin-Reihe werden zum Färben von synthetischen Fasermaterialien verwendet, zeigen jedoch noch unbefriedigende anwendungstechnische Eigenschaften.

Aus DE-C 955 178 ist ein Verfahren Zur Herstellung von Isoindolenin-Farbstoffen durch Umselzung von aromatischen o-Dinitrilen mit Amino thiazolen bekannt.

Es wurden nun Farbstoffe der Formel (I) 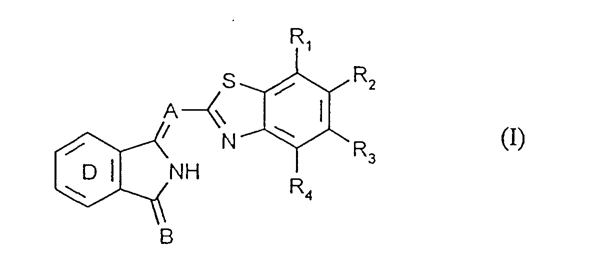 gefunden, worin
- A: für N oder einen Cyanmethylenrest,
- B: für einen Rest der Formel C(CN)COOR₅ oder N-R₆ steht, mit der Maβgabe, dass A für einen Cyanmethylenrest steht, falls B für N-R₆ steht,
- R₁ bis R₄: unabhängig voneinander Wasserstoff, Halogen, gegebenenfalls substituiertes C₁-C₈-Alkyl oder C₅-C₆-Cycloalkyl, gegebenenfalls durch Sauerstoff unterbrochenes C₁-C₁₀-Alkoxy, gegebenenfalls substituiertes C₆-C₁₀-Aryloxy, CF₃, oder gegebenenfalls substituiertes Dialkylamin bedeuten oder jeweils zwei benachbarte R₁ bis R₄-Reste zusammen mit den aromatischen Ring C-Atomen einen annelierten Benzol- oder Naphthalinring bilden, der gegebenenfalls weiter substituiert sein kann, wobei als Substituenten beispielsweise Halogen oder C₁-C₄-Alkyl genannt werden können,
- R₅: für einen gegebenenfalls substituierten, gesättigten oder ungesättigten C₁-C₂₀-Alkylrest, C₆-C₁₀-Aryl-C₁-C₁₀-alkyl- oder Hetarylalkylrest steht, wobei der Alkylrest gegebenenfalls durch Sauerstoffatome unterbrochen ist,
- R₆: gegebenenfalls substituiertes C₅-C₆-Cycloalkyl, C₅-C₆-Cycloalkyl-C₁-C₈-alkyl, C₆-C₁₀-Aryl-C₁-C₁₀-alkyl oder C₁-C₂₀-Alkyl bedeutet, wobei letzteres gegebenenfalls durch Sauerstoffatome unterbrochen ist und
der Ring D unsubstituiert ist oder wenigstens einen Substituenten trägt, welcher gegebenenfalls, zusammen mit einem weiteren Substituenten in o-Stellung und den Ring-C-Atomen, einen annelierten Benzol- oder Naphthalinring bildet, mit Ausnahme des Farbstoffs der Formel 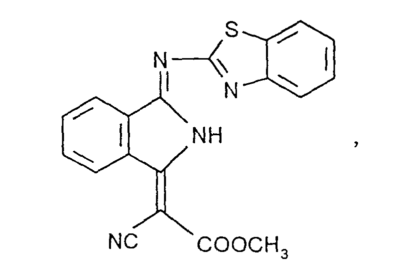 der aus EP-A 61 094 bekannt ist.

Geeignete Reste R₁ bis R₄ sind z.B. Wasserstoff, Chlor, Brom, Methyl, Ethyl, iso-Propyl, tert.-Butyl, Methoxy, Ethoxy, n-Propoxy, n-Butoxy, Methoxy-ethyl, Methoxy-ethoxy-ethyl, Ethoxy-ethyl, Ethoxy-ethoxy-ethyl, Butoxy-ethyl, Phenoxy, 2-Methyl-phenoxy, 3-Methyl-phenoxy, Phenoxy-ethoxy, 4-Methyl-phenoxy, Dimethylamino, Diethylamino, Bis-(2-cyan-ethyl)-amino.

Geeignete Reste R₅ sind z.B.: Methyl, Ethyl, n-Propyl, iso-Propyl, Allyl, n-Butyl, n-Pentyl, n-Hexyl, n-Heptyl, n-Octyl, n-Decyl, 2-Methoxy-ethyl, 2-Ethoxy-ethyl, 2-Isopropoxy-ethyl, 2-Butoxy-ethyl, 2-Allyloxy-ethyl, 2-(2-Methoxy-ethoxy)-ethyl, 2-(2-Ethoxy-ethoxy)-ethyl, 2-(2-Methoxy-ethoxy)-ethyl, 2-Cyan-ethyl, 2-(Cyan-ethoxy)-ethyl, 4-(2-Cyan-ethoxy)-butyl, 2-Ethyl-hexyl, Benzyl, Phenylethyl. 3-Phenylpropyl, Phenoxy-ethyl, Furfuryl. Als verzweigte Reste R₅ kommen vorzugsweise solche mit einer Methylseitenkette in Frage wie z.B.: iso-Butyl, tert.-Butyl, iso-Pentyl, 1-Methoxy-2-propanol, 1-Ethoxy-2-propanol.

Geeignete Reste R₆ sind z.B.: Methyl, Ethyl, n-Propyl, iso-Propyl, Allyl, n-Butyl, n-Pentyl, n-Hexyl, n-Heptyl, n-Octyl, n-Decyl, 2-Ethyl-hexyl, 2-Methoxy-ethyl, 2-Ethoxy-ethyl, 3-Methoxy-propyl, 3-Ethoxy-propyl, 3-Butoxy-propyl, 3-Phenoxypropyl, 3-(2-Phenoxy-ethoxy)-propyl, Cyclohexyl, Cyclohexylmethyl, Benzyl, 2-Phenyl-ethyl.

Bevorzugt sind Farbstoffe der Formel (I), worin
- R₁ und R₂: unabhängig voneinander Wasserstoff, Cl, Br, Methyl, Ethyl, n-Propyl, Isopropyl, n-Butyl, iso-Butyl, tert.-Butyl, Cyclohexyl, gegebenenfalls durch 1 bis 2 Sauerstoffe unterbrochenes C₁-C₁₀-Alkoxy, gegebenenfalls substituiertes Phenoxy, CF₃ oder eine Di(C₁-C₄)-Alkylaminogruppe bedeuten,
- R₃ und R₄: die Bedeutung von R₁ und R₂ haben oder zusammen mit den Ring-C-Atomen einen annelierten Benzolring bilden,
- R₅: einen gegebenenfalls durch Cl, CN oder gegebenenfalls substituiertes Phenoxy, substituiertes und gegebenenfalls durch 1 bis 2 Sauerstoffatome unterbrochenes C₁-C₁₂-Alkyl, C₆-C₁₀-Aryl-C₁-C₁₀-Alkyl oder Hetarylalkyl,
- R₆: gegebenenfalls durch gegebenenfalls substituiertes Phenoxy, substituiertes gesättigtes oder ungesättigtes C₁-C₁₂-Alkyl bedeutet, das gegebenenfalls durch 1 bis 2 Sauerstoffe unterbrochen ist und
Ring D unsubstituiert oder durch CN, Halogenatome, insbesondere 1 bis 4 Cl-Atomen, 1 bis 2 C₁-C₁₀-Alkylreste und/oder 1 bis 2 C₁-C₁₀-Alkoxyreste oder einen Phenylrest, substituiert ist, die gegebenenfalls jeweils durch 1 bis 2 Sauerstoffatome unterbrochen sind. Insbesondere ist der Ring D jedoch unsubstituiert.

Besonders bevorzugte Farbstoffe der Formel (I) sind solche der Formel (II) 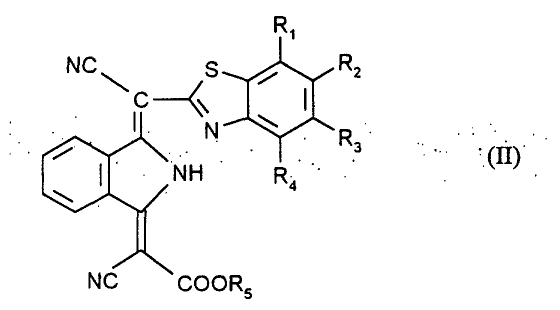 worin R₁ bis R₅ die obige Bedeutung haben, vorzugsweise steht
- R₁ bis R₄: unabhängig voneinander für Wasserstoff, Chlor, Methyl, Ethyl, iso-Propyl, tert.-Butyl, Cyclohexyl, Methoxy, Ethoxy, n-Propoxy, n-Butoxy, Methoxy-ethyl, Ethoxy-ethyl, Butoxy-ethyl oder Phenoxy und
- R₅: für n-Butyl, iso-Butyl, n- und iso-Pentyl, Hexyl, Octyl, 2-Ethyl-hexyl, Methoxy-ethyl, Ethoxy-ethyl, Butoxy-ethyl, Butoxy-ethoxy-ethyl.

Weiterhin bevorzugt sind Farbstoffe der Formel (I), die der Formel (III) entsprechen 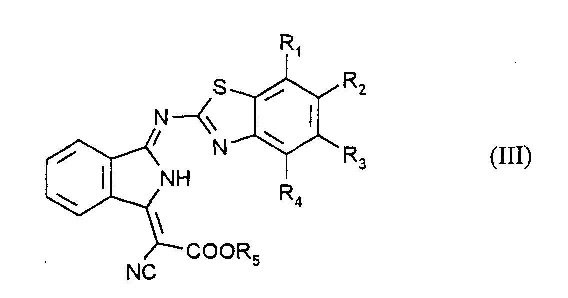 worin R₁ bis R₅ die oben angegebene Bedeutung besitzt, vorzugsweise steht
- R₁ bis R₄: unabhängig voneinander für Wasserstoff, Chlor, Methyl, Ethyl, iso-Propyl, tert.-Butyl, Cyclohexyl, Methoxy, Ethoxy, n-Propoxy, n-Butoxy, Methoxy-ethyl, Ethoxy-ethyl, Butoxy-ethyl oder Phenoxy und
- R₅: für Methyl, Ethyl, Propyl, iso-Propyl, Allyl, n-Butyl, iso-Butyl, n- und iso-Pentyl, Hexyl, Octyl, 2-Ethyl-hexyl, Methoxy-ethyl, Ethoxy-ethyl, Butoxyethyl, Butoxy-ethoxy-ethyl.

Ebenfalls bevorzugt sind Farbstoffe der Formel (I), die der Formel (IV) entsprechen 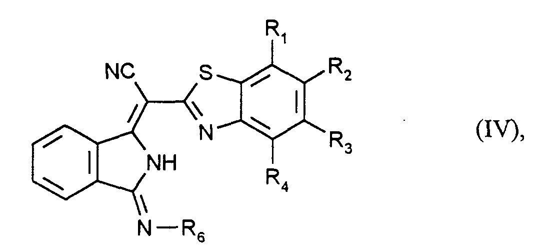 worin R₁ bis R₄ und R₆ die oben angegebene Bedeutung haben, vorzugsweise steht
- R₁ bis R₄: unabhängig voneinander für Wasserstoff, Chlor, Methyl, iso-Propyl, tert.-Butyl, Cyclohexyl, Methoxy, Ethoxy, n-Propoxy, n-Butoxy, Methoxyethyl, Ethoxy-ethyl, Butoxy-ethyl oder Phenoxy und
- R₆: für Methyl, Ethyl, Propyl, iso-Propyl, Allyl, n-Butyl, iso-Butyl, n- und iso-Pentyl, Hexyl, Octyl, 2-Ethyl-hexyl, Cyclohexyl, Methoxy-propyl, Ethoxypropyl, 2-Phenoxy-ethyl, 3-Phenoxypropyl, 2-Phenoxy-ethoxy-propyl, Phenylethyl.

Weiterhin betrifft die Erfindung Verfahren zur Herstellung von Verbindungen der Formel (I). Die Summe aller von der Formel (I) erfaßten Farbstoffe ist prinzipiell durch ein einheitliches Verfahren herstellbar (siehe z.B. DE-A-1 670 748, DE-A-2 628 409, EP-A-62 614, EP-A-510 436). Eine solche Standardprozedur führt jedoch bei manchen Verbindungen der Formel (I) zu nur mäßigem Erfolg. Daher wurden die Teilformeln (II), (III) und (IV) 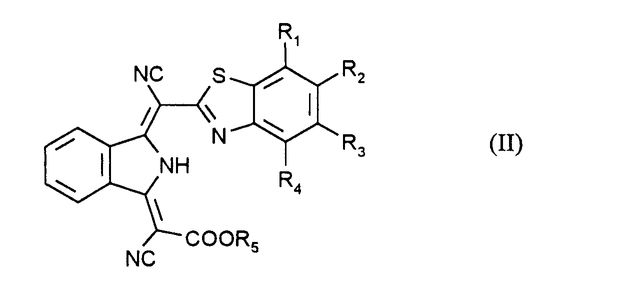 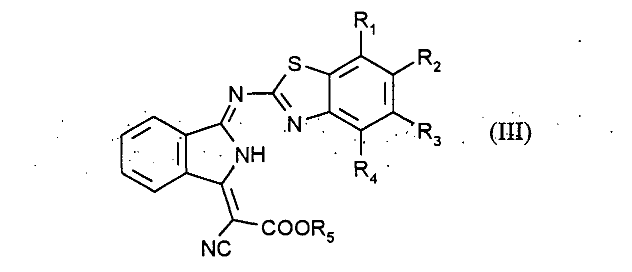 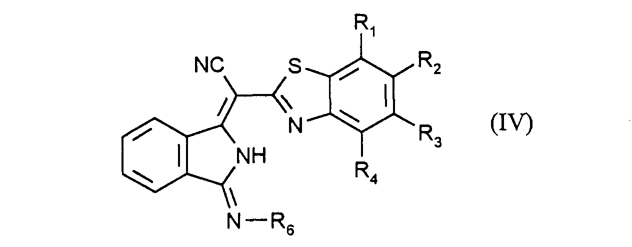 aufgestellt, worin die Reste R₁ bis R₆ die oben angegebene Bedeutung besitzen und für die jeweils eine etwas andere Verfahrensweise zu den besten Ergebnissen führt.

Das Verfahren zur Herstellung von Verbindungen der Formel (II) ist dadurch gekennzeichnet, daß man ein Aminoisoindolenin der Formel (VII) 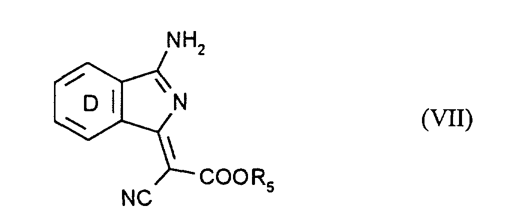 mit einem 2-Cyanmethyl-benzthiazol der Formel (VIII) 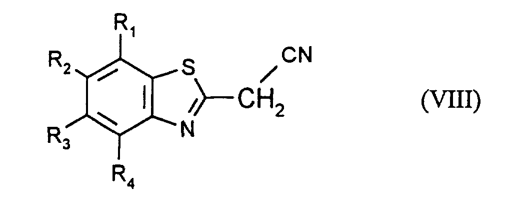 oder ein Aminoisoindolenin der Formel (IX) 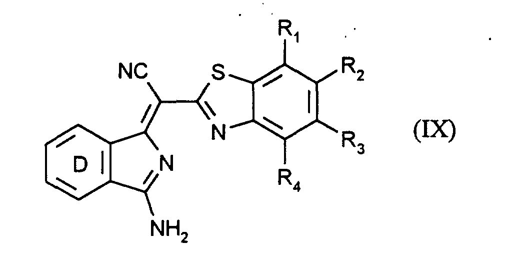 mit einem Cyanessigsäureester der Formel (VI)

NC-CH₂-COOR₅ (VI)

kondensiert, worin

R₁ bis R₆ und D die obengenannte Bedeutung besitzen, wobei die ausgeschlossene Verbindung ebenfalls ausgeschlossen ist.

In einer bevorzugten Ausführungsform erfolgt das erfindungsgemäße Verfahren zur Herstellung von Verbindungen der Formel (II) in einem polaren, insbesondere hydrophilen organischen Lösungsmittel.

Als polare Lösungsmittel sind beispielsweise zu nennen: Amide wie Dimethylformamid, Formamid, Dimethylacetamid, N-Methylpyrrolidon, ferner Dimethylsulfoxid, Acetonitril, Essigsäure oder Alkohol, wobei vorzugsweise der Alkohol eingesetzt wird, der auch in den Essigsäureestern der Formel (VI) als alkoholische Komponente benutzt wird. Darüber hinaus können auch Mischungen dieser Lösungsmittel Verwendung finden.

Besonders bevorzugt bei dieser Verfahrensweise ist auch der Zusatz einer organischen Säure. Dieser führt zu einer Beschleunigung der Reaktion, einer verbesserten Kristallinität und einer höheren Ausbeute. Als geeignete organische Säuren sind z.B. niedrige aliphatische, gesättigte oder ungesättigte Mono- oder Dicarbonsäuren wie z.B. Ameisensäure, Essigsäure, Propionsäure, Buttersäure, Oxalsäure, Fumarsäure, Maleinsäure, Bernsteinsäure, Glutarsäure, Adipinsäure, Äpfelsäure, Milchsäure, Zitronensäure, aber auch aromatische Säuren, wie beispielsweise Benzoesäure und Phthalsäure zu nennen. Die Säuren werden in Mengen von 0,2 bis 3 Mol-Äquivalenten, vorzugsweise 1 bis 2 Mol-Äquivalenten, jeweils bezogen auf das einzusetzende Ausgangsaminoisoindolin der Formel (VII) oder (IX) zugegeben. Aber auch höhere Mengen an Säure können eingesetzt werden bevorzugt dann, wenn die Säure gleichzeitig als Lösungsmittel dienen soll, beispielsweise Essigsäure.

In einer besonders bevorzugten Ausführungsform des Verfahrens zur Herstellung von Verbindungen der Formel (II) wird die Reaktion in Wasser oder einem wasserhaltigen Medium durchgeführt. Neben dem Wasser können dabei auch organische Lösungsmittel zugegen sein, und zwar bevorzugt solche, die mit Wasser ganz oder teilweise mischbar sind wie z.B. Alkohole, bevorzugt die Alkohole, die dem Rest R₅ zugrunde liegen, Ketone wie z.B. Aceton, Methylethylketon, Cyclohexanon, Ether wie Tetrahydrofuran und Dioxan, Dimethylformamid, N-Methylpyrrolidon und andere. Es können jedoch auch mit Wasser nicht mischbare Lösungsmittel dem wäßrigen Reaktionsmedium zugesetzt werden, um z.B. die Kristallinität zu verbessern und besondere Kristallformen zu erzielen. Die organischen Lösungsmittel können von Anfang an zugegen sein oder aber auch erst im Verlauf der Reaktion zugegeben werden. Auch hierbei kann ein Zusatz einer organischen Säure sich vorteilhaft auswirken und zu einer Beschleunigung der Reaktion, einer verbesserten Kristallinität sowie einer höheren Ausbeute führen.

Das Arbeiten mit Wasser bzw. wasserhaltigen Reaktionsmedien erleichtert die Isolierung der Farbstoffe und vermeidet die Aufarbeitung von größeren Mengen an organischen Lösungsmitteln. Bevorzugt wird bei dieser Verfahrensvariante mit Wassergehalten von 20 bis 100 %, insbesondere 50 bis 100 % gearbeitet (bezogen auf die Menge des verwendeten Reaktionsmediums).

Verwendet man Wasser oder ein überwiegend wäßriges Medium als Reaktionsmedium, so werden zweckmäßigerweise oberflächenaktive Substanzen wie Tenside, Dispergiermittel, Emulgatoren und Netzmittel zugegeben. In Betracht kommen die bekannten nichtionogenen, anionischen und kationischen Hilfsmittel. Solche Verbindungen sind z.B. Salze von Alkylbenzolsulfonsäuren, Alkylphenolsulfonsäuren, Alkylnaphthalinsulfonsäuren, Kondensationsprodukte aus Phenolsulfonsäuren, Formaldehyd und Harnstoff, Ligninsulfonate, Additionsprodukte von Ethylen- und Propylenoxid an Alkanole, Alkandiole, Phenole, Carbonsäuren, Amine, Carbonsäureamide und ihre Schwefelsäurehalbester, wobei auch Mischungen dieser Verbindungen eingesetzt werden können. Besonders bevorzugt sind jedoch Ligninsulfonate, wie z.B. Kraftlignine beispielsweise vom Typ Reax® der Firma Westvaco oder Sulfitlignine beispielsweise vom Typ Ufoxane® der Firma Borregaard oder Gemische davon.

Die Kondensationsreaktionen können je nach Art der verwendeten Einsatzstoffe und Reaktionsmedium in einem weiten Temperaturbereich durchgeführt werden, bevorzugt sind jedoch Temperaturen zwischen 40°C und 120°C.

Ebenfalls ganz besonders bevorzugt ist ein Verfahren zur Herstellung von Verbindungen der Formel (II), das dadurch gekennzeichnet ist, daß die eingesetzte Aminoisoindolenin-Verbindung der Formel (VII) bzw. (IX) durch Kondensation von Amino-Imino-Isoindolenin der Formel (V) 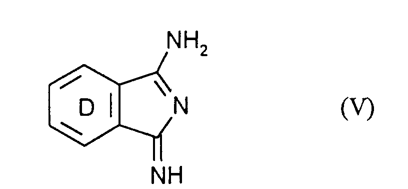 mit den Methinverbindungen der Formel (VI) bzw. (VIII) hergestellt wird. Dieses ganz besonders bevorzugte Verfahren läßt sich in dem folgenden Reaktionsschema darstellen:

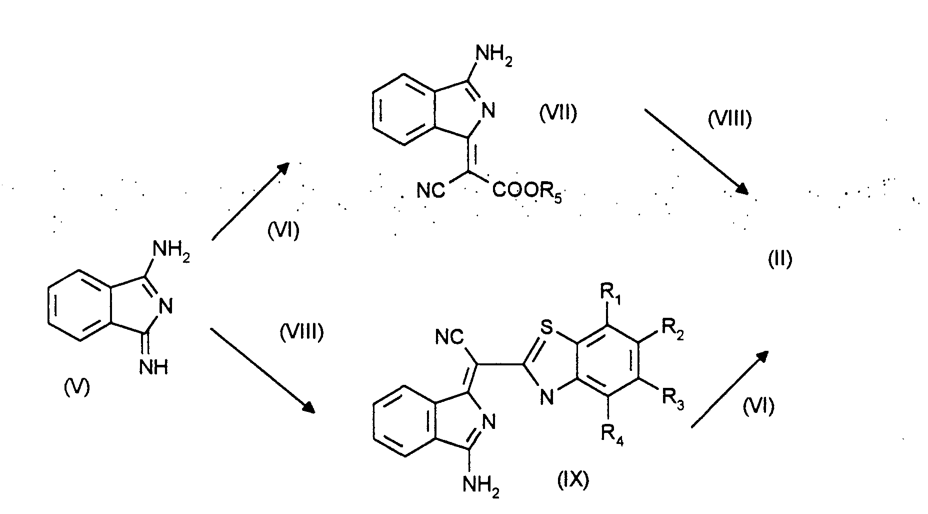

Bevorzugt ist demnach das obige Verfahren zur Herstellung von Farbstoffen der Formel (II), dadurch gekennzeichnet, daß das eingesetzte Amino-isoindolenin der Formel (VII) durch Umsetzung der Amino-Imino-Isoindolin-Verbindung der Formel (V) 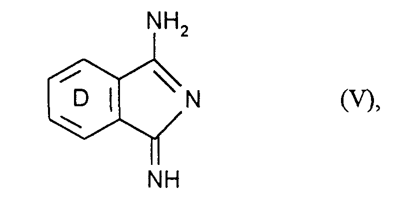 worin D die obige Bedeutung hat,
mit einem Cyanessigsäureester der Formel (VI)

NC-CH₂-COOR₅ (VI),

worin R₅ die obige Bedeutung hat,
erhalten wird, oder das eingesetzte Aminoisoindolenin der Formel (IX) durch Umsetzung von Amino-Imino-Isoindolin der Formel (V) mit einem 2-Cyanmethylbenzthiazol der Formel (VIII) 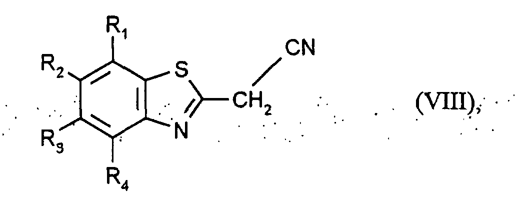 worin R₁ bis R₄ die obige Bedeutung haben, erhalten wird.

Diese erfindungsgemäße Verfahrenssequenz ausgehend von den Amino-Imino-Isoindolin-Verbindungen der Formel (V) zu Farbstoffen der Formel (II) kann vorzugsweise ebenfalls in Wasser oder in einem wasserhaltigen Medium durchgeführt werden. Bei dieser Verfahrensweise ist insbesondere auch eine Eintopfreaktion möglich, d.h. ohne Zwischenisolierung der monokondensierten Amino-Isoindolenin-Verbindungen der Formel (VII) bzw. (IX).

Das Verfahren zur Herstellung von Verbindungen der Formel (III) ist dadurch gekennzeichnet, daß Verbindungen der Formel (X) 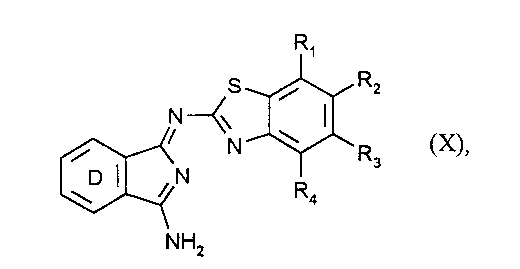 worin D und R₁ bis R₄ die obige Bedeutung haben,
mit Cyanessigsäureestern der Formel (VI)

NC-CH₂-COOR₅ (VI),

worin R₅ die obige Bedeutung hat,
kondensiert werden. Die bevorzugten Reaktionsbedingungen dieses Herstellungsverfahrens entsprechen denen für die Herstellung von Verbindungen der Formel (II). Auch hier wurde überraschenderweise gefunden, daß die Kondensationsreaktion unter sonst gleichen Bedingungen besonders vorteilhaft in Wasser oder in Mischungen von Wasser mit organischen Lösungsmitteln durchgeführt werden kann.

Verbindungen der Formel (X) können auf verschiedene Weise hergestellt werden. So lassen sich Amino-Imino-Isoindoleninen der Formel (V) 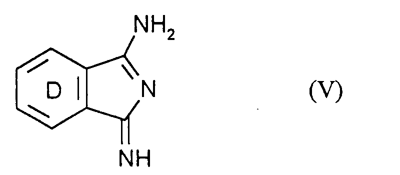 mit 2-Amino-benzthiazolen der Formel (XI) 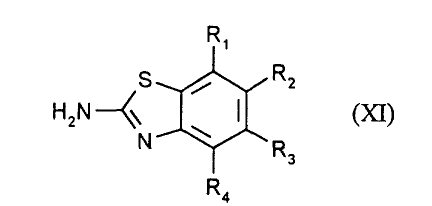 kondensieren, wobei R₁ bis R₄ und D die obige Bedeutung besitzen. Die Kondensation von (V) und (XI) zu (X) kann durch Erhitzen der Komponenten in einem organischen Lösungsmittel durchgeführt werden, wobei als Lösungsmittel beispielsweise Amide, wie Formamid, Dimethylformamid, Dimethylacetamid und N-Methylpyrrolidon und bevorzugt Alkohole, insbesondere niedere Alkohole wie Methanol, Ethanol, n-Propanol und iso-Propanol eingesetzt werden.

Ein weiteres Verfahren zur Herstellung von Verbindungen der Formel (X) wurde gefunden, das dadurch gekennzeichnet ist, daß man 2-Aminobenzthiazole der Formel (XI) 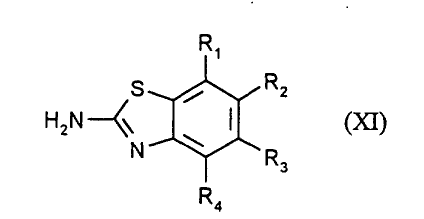 mit Phthalsäuredinitrilen der Formel (XII) 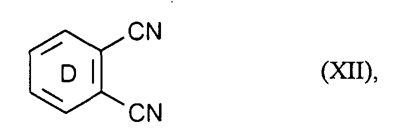 wobei R₁ bis R₄ und D die obige Bedeutung besitzen, in Gegenwart einer Base umsetzt.

Diese Additionsreaktion kann in einem organischen Lösungsmittel durchgeführt werden, wobei als Lösungsmittel Amide, wie beispielsweise Formamid, Dimethylformamid, Dimethylacetamid und N-Methylpyrrolidon und bevorzugt Alkohole und ganz besonders bevorzugt niedere Alkohole wie Methanol, Ethanol, n-Propanol und iso-Propanol verwendet werden können. Diese Reaktion wird vorzugsweise durch Alkoholate wie Natriummethylat, Natriumethylat, Kalium-tert.-Butylat katalysiert. Die Alkoholatmenge kann in weiten Grenzen variiert werden, bevorzugt sind jedoch Mengen zwischen 0,5 und 1 Mol-Äquivalent, bezogen auf die eingesetzte Menge an (XII).

Geeignete Reaktionstemperaturen liegen zwischen 0 und 100°C, bevorzugt zwischen 20 und 60°C. Der Vorteil dieses Verfahrens zur Herstellung von Verbindungen der Formel (X) ist der, daß das Aminoiminoisoindolenin, das normalerweise ebenfalls aus Phthalsäuredinitril hergestellt wird, nicht zuvor hergestellt werden muß. Somit werden höhere Ausbeuten, insbesondere höhere Raumzeitausbeuten erzielt, zumal diese Reaktion sehr schnell abläuft.

Das Verfahren zur Herstellung von Verbindungen der Formel (IV) ist dadurch gekennzeichnet, daß Aminoisoindolenine der Formel (IX) 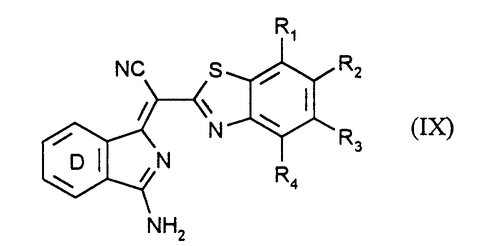 mit Aminen der Formel R₆-NH₂ umgesetzt werden, wobei D, R₁ bis R₄ die obige Bedeutung haben. In einer bevorzugten Ausführungsform dieses Verfahrens werden die bevorzugten Bedingungen gewählt, wie sie zur Herstellung von Verbindungen der Formel (II) beschrieben sind.

Ein bevorzugtes Verfahren zur Herstellung von Verbindungen der Formel (IX) ist bereits oben beschrieben.

Als weiteres Verfahren zur Herstellung von Verbindungen der Formel (IV) ist die Umsetzung zu nennen, die dadurch gekennzeichnet ist, daß man Verbindungen der Formel (XIII) 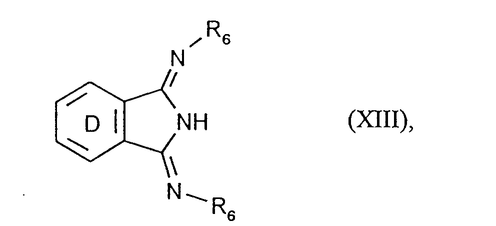 worin R₆ und D die obige Bedeutung besitzen,
mit Verbindungen der Formel (VIII) 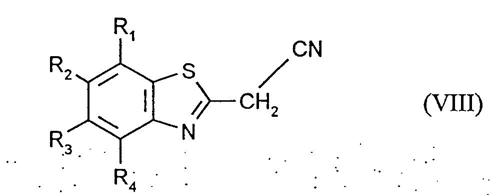 worin R₁ bis R₄ die obige Bedeutung besitzen,
umsetzt. Bevorzugte Reaktionsbedingungen für diese Umsetzungen sind ebenfalls die, die schon für die Herstellung von Verbindungen der Formel (II) und (IV) angegeben worden sind.

Die Erfindung betrifft weiterhin die Verwendung von Farbstoffen der Formel (I) zum Färben von vollsynthetischen oder halbsynthetischen, hochmolekularen Stoffen. Besonders geeignet sind die nenen Farbstoffe zum Farben oder Bedrucken von synthetischen Fasermaterialien, insbesondere solchen aus aromatischen Polyestern und/oder Celluloseacetaten. Die dabei erhaltenen Färbungen besitzen eine hohe Farbstärke und zeigen eine überragende Lichtechtheit, insbesondere eine hohe Heißlichtechtheit und sind daher besonders zum Färben und Bedrucken von Textilmaterialien für die Automobilindustrie sowie für das Färben von sogenannten Microfasern geeignet.

Farbstoffe der Formel (I) eignen sich auch hervorragend für das sogenannte Thermo-Transfer-Printing auf textilen und nichttextilen Substraten z.B. nach dem D2T2 (Dye Diffusion Thermo Transfer)-Prozeß für die Bildaufzeichnung. Weiterhin können die Farbstoffe zum Massefärben von Kunststoffen zB. von Polyethylenen, Polypropylenen, Polystyrol, Polycarbonaten sowie von Kunststoffblends wie z.B. ABS verwendet werden. Die Farbstoffe, insbesondere die der Formeln (II) und (IV), fluoreszieren teilweise und eignen sich daher auch als Fluoreszenzfarbstoffe.

Textilmaterialien aus Polyester können mit den erfindungsgemäßen Farbstoffen nach Art einer Spinnfärbung gefärbt werden, bevorzugt jedoch aus wäßriger Suspension. Hierzu werden die Farbstoffe auf allgemein bekannte Weise zu Färbepräparaten verarbeitet, z.B. durch Mahlen in Wasser in Gegenwart von Dispergier- und/oder Füllmitteln. Mit den gegebenenfalls im Vakuum oder durch Zerstäuben getrockneten Präparaten kann man nach Zugabe von Wasser in sogenannter kurzer oder langer Flotte färben, klotzen oder bedrucken.

Insbesondere zum Färben von Polyester eignen sich auch Mischungen von mindestens zwei Farbstoffen der Formeln (I) bis (IV), wodurch unter Umständen das Zieh- und Aufbauvermögen der Farbstoffe, ihre Dispergierbarkeit sowie das Egalisiervermögen verbessert werden können.

Als weiterer Vorteil hat sich bei Verwendung von Farbstoff-Mischungen gegenüber Einzelfarbstoffen eine verbesserte Färbebadstabilität gezeigt. Bei nicht ausreichender Färbebadstabilität kann der oder die Farbstoffe unter Färbebedingungen aus dem Färbebad ausfallen, was zu unegalen Färbungen führt.

Dabei sind insbesondere Mischkristalle von mindestens zwei Farbstoffen der Formeln (I) bis (IV) bevorzugt, wie sie beispielsweise bei einer gemeinsamen Synthese entstehen können.

Die Farbstoffmischungen können nach verschiedenen Verfahren hergestellt werden. Beispielsweise durch:
a) Abmischen der separat hergestellten und formierten Einzelfarbstoffe,
b) gemeinsame Formierung der separat hergestellten Einzelfarbstoffe oder
c) gemeinsame Synthese von Mischungen der Farbstoffe aus Mischungen unterschiedlicher Vorprodukte, soweit dies möglich ist.

Das Mischen der Farbstoffe erfolgt zweckmäßigerweise in geeigneten Mühlen, beispielsweise Kugel- oder Sandmühlen. Einzeln formierte Farbstoffe können aber auch durch Einrühren in Färbeflotten gemischt werden. Zur Herstellung bzw. Verbesserung des Dispersionsgrades von Einzelfarbstoffen oder Mischungen wird der zu mahlenden Mischung oder der Reaktionsmischung vorzugsweise ein oder mehrere Dispergiermittel zugesetzt. Selbstverständlich kann die Teilchengröße der Farbstoffpartikel durch eine Mahlbehandlung z.B. Naßperlmahlung, sei es während der Synthese oder im Anschluß daran, entsprechend beeinflußt und auf einen gewünschten Wert eingestellt werden. Als Dispergiermittel zur Formierung der erfindungsgemäßen Einzelfarbstoffe sowie Mischungen von mindestens zwei Farbstoffen der Formeln (I) kommen insbesondere anionische und/oder nichtionogene in Frage. Bevorzugt sind dabei die anionischen Dispergiermittel und besonders bevorzugt ist eine Mischung aus anionischen und nichtionogenen Dispergiermitteln.

Geeignete anionische Dispergiermittel sind insbesondere Kondensationsprodukte von aromatischen Sulfonsäuren mit Formaldehyd, wie Kondensationsprodukte aus Formaldehyd und Alkylnaphthalinsulfonsäuren oder aus Formaldehyd, Naphthalinsulfonsäuren und Benzolsulfonsäuren, Kondensationsprodukte aus gegebenenfalls substituiertem Phenol mit Formaldehyd und Natriumbisulfit. Weiterhin kommen vor allem Ligninsulfonate in Betracht, z.B. solche, die nach dem Sulfit- oder Kraft-Verfahren gewonnen werden. Vorzugsweise handelt es sich um Produkte die zum Teil hydrolysiert, oxidiert, propoxyliert, sulfoniert, sulfomethyliert oder desulfoniert und nach bekannten Verfahren fraktioniert werden, z.B. nach dem Molekulargewicht oder nach dem Sulfonierungsgrad. Auch Mischungen aus Sulfit- und Kraftligninsulfonaten sind gut wirksam. Besonders geeignet sind Ligninsulfonate mit einem durchschnittlichen Molekulargewicht zwischen 1 000 und 100 000, einem Gehalt an aktivem Ligninsulfonat von mindestens 80 % und vorzugsweise mit niedrigem Gehalt an mehrwertigen Kationen. Der Sulfonierungsgrad kann in weiten Grenzen variieren.

Als nichtionische Dispergiermittel kommen beispielsweise in Frage: Umsetzungsprodukte von Alkylenoxiden mit alkylierbaren Verbindungen, wie z.B. Fettalkoholen, Fettaminen, Fettsäuren, Phenolen, Alkylphenolen, Arylalkylphenolen, Carbonsäureamiden und Harzsäuren. Hierbei handelt es sich z.B. um Ethylenoxidaddukte aus der Klasse der Umsetzungsprodukte von Ethylenoxid mit:
a) gesättigten und/oder ungesättigten Fettalkoholen mit 6 bis 20 C-Atomen oder
b) Alkylphenolen mit 4 bis 12 C-Atomen im Alkylrest oder
c) gesättigten und/oder ungesättigten Fettaminen mit 14 bis 20 C-Atomen oder
d) gesättigten und/oder ungesättigten Fettsäuren mit 14 bis 20 C-Atomen oder
e) hydrierte und/oder unhydrierte Harzsäuren.

Als Ethylenoxid-Addukte kommen insbesondere die unter. a) bis e) genannten alkylierbaren Verbindungen mit 5 bis 30 Mol Ethylenoxid in Frage.

Die Farbstoffpräparationen der Einzelfarbstoffe der Formel (I) oder deren Mischungen enthalten im allgemeinen:
10-60 Gew.-% Einzelfarbstoff/Mischung
10-80 Gew.-% anionisches Dispergiermittel
0 bis 15 Gew.-% nichtionogenes Dispergiermittel und gegebenenfalls weitere Zusätze wie Netzmittel, Entschäumer, Entstaubungsmittel sowie weitere Hilfsmittel.

Geeignete Netzmittel sind beispielsweise C₆-C₁₀-Alkyl-Phosphate oder Additionsprodukte aus C₆-C₁₈-Fettalkoholen und Ethylen- und/oder Propylenoxid oder Gemische solcher Alkoxylierungsprodukte.

Geeignete Entschäumer sind beispielsweise Tributylphosphat oder Tertiär-Acetylen-Glykol.

Geeignete Entstaubungsmittel sind beispielsweise solche auf Mineralölbasis.

Unter weiteren Hilfsmitteln werden beispielsweise Fungizide, Antrocknungsverhinderer usw. verstanden.

Bevorzugte Farbstoffpräparationen enthalten
10 bis 50 Gew.-% Einzelfarbstoff bzw. Farbstoffmischung
10 bis 80 Gew.-% Ligninsulfonate, insbesondere Kraft- und Sulfitlignine
0 bis 20 Gew.-% eines Kondensationsproduktes aus Naphthalinsulfonsäure und Formaldehyd,

0 bis 10 Gew.-% nichtionogenes Dispergiermittel, insbesondere Additionsprodukte von Harzsäuren und Ethylenoxid und/oder Propylenoxid,
0,1 bis 1,5 Gew.-% Netzmittel,
0,1 bis 1 Gew.-% Entschäumer,
0,2 bis 1,5 Gew.-% Entstaubungsmittel, jeweils bezogen auf die gesamte Präparation.

Bei den Farbstoffmischungen sind solche bevorzugt, die mindestens zwei Farbstoffe der Formeln (I) enthalten, bei dem der Anteil eines Farbstoffs, bezogen auf die Mischung, vorzugsweise 10-90 Gew.-% und der Anteil des anderen Farbstoffes bei vorzugsweise 90-10 Gew.-% liegt. Besonders bevorzugte Mischungen sind solche, bei denen der Anteil eines Farbstoffs, bezogen auf die Mischung bei 60 bis 90 % und der Anteil des anderen Farbstoffes bei 10 bis 40 % liegt.

Ganz besonders bevorzugt handelt es sich bei den Farbstoff-mischungen um solche, die mindestens zwei Farbstoffe der Formeln (II) oder (III) oder mindestens einen Farbstoff der Formel (II) und mindestens einen Farbstoff der Formel (III) enthalten. Darunter sind insbesondere Mischungen zu verstehen, die mindestens zwei Farbstoffe der Formel (II) enthalten, die sich durch einen oder mehrere Reste R₁ bis R₅, bevorzugt jedoch lediglich durch den Rest R₅ unterscheiden.

Gleichbedeutend als bevorzugt gelten Farbstoffmischungen, die mindestens zwei Farbstoffe der Formel (III) enthalten, die sich durch einen oder mehrere Reste R₁ bis R₅, bevorzugt jedoch lediglich durch den Rest R₅ unterscheiden.

Besonders bevorzugte Mischungen enthalten mindestens zwei Farbstoffe, bei denen der Rest R₅ in dem einen Farbstoff für C₁-C₂₀-Alkyl steht und in dem zweiten Farbstoff für ein durch Sauerstoffatome unterbrochenen C₁-C₂₀-Alkylrest, insbesondere ein Alkoxyalkylrest, steht. Bevorzugte Alkylreste sind z.B.: Ethyl, Propyl, n-Butyl, Isobutyl, n-Pentyl und n-Hexyl. Bevorzugte Alkoxyalkylreste sind z.B. Ethoxy-ethyl, Propyl-ethyl, iso-Propoxyethyl und Butoxyethyl.

Das Färben des genannten Fasergutes mit den erfindungsgemäßen Einzelfarbstoffen, Farbstoffmischungen sowie den jeweiligen Farbstoffpräparationen erfolgt in an sich bekannter Weise, vorzugsweise aus wäßriger Suspension, gegebenenfalls in Gegenwart von Carriern im allgemeinen bei 80 bis 110°C nach dem Ausziehverfahren oder nach dem HT-Verfahren im Färbeautoklav bei 110 bis 140°C. Man erhält auf diese Weise sehr farbstarke gelbe bis orange Färbungen mit sehr guten Echtheitseigenschaften. In den bei den obigen Applikationen eingesetzten Färbeflotten sollen die erfindungsgemäßen Einzelfarbstoffpräparationen bzw. die Präparationen der Farbstoffmischungen in möglichst feiner Verteilung vorliegen. Die Feinverteilung der Farbstoffe erfolgt in an sich bekannter Weise dadurch, daß man die Einzelfarbstoffe bzw. die Farbstoffmischungen zusammen mit Dispergiermitteln in einem flüssigen Medium, vorzugsweise in Wasser, aufschlämmt und die Mischung der Einwirkung von Scherkräften aussetzt, wobei die ursprünglich vorhandenen Farbstoffteilchen mechanisch soweit zerkleinert werden, daß eine optimale spezifische Oberfläche erreicht wird und die Sedimentation des Farbstoffs möglichst vermieden wird. Die Teilchengröße der Farbstoffe liegen im allgemeinen zwischen 0,1 und 5 µm, vorzugsweise zwischen 0,5-0,1 µm. Die bei dem Mahlvorgang mitverwendeten Dispergiermittel können die bereits im Zusammenhang mit der Synthese und der Herstellung der Mischung genannten nichtionischen oder anionischen Dispergiermittel sein.

Für die meisten Anwendungsbereiche werden feste Präparationen (Pulver- bzw. Granulatpräparationen) bevorzugt.

Ein bevorzugtes Herstellungsverfahren für feste Farbstoffzubereitungen besteht darin, daß den oben beschriebenen flüssigen Farbstoffdispersionen die Flüssigkeit entzogen wird, z.B. durch Vakuumtrocknung, Gefriertrocknung, durch Trocknung auf Walzentrocknern, vorzugsweise aber durch Sprühtrocknung.

Zur Herstellung der erfindungsgemäßen Einzelfarbstoffen bzw. Farbstoff-mischungen in feinverteilter Form kann folgendermaßen vorgegangen werden: beispielsweise werden 10 bis 50 Teile eines erfindungsgemäßen Einzelfarbstoffes oder einer Farbstoffmischung mit 10 bis 80 Teilen Ligninsulfonat, 20 bis 0 Teilen Kondensationsprodukt aus Naphthalinsulfonsäuren und Formaldehyd, 10 bis 0 Teilen nichtionogenes Dispergiermittel, 0,1 bis 1,5 Teilen Netzmittel, 0,1 bis 1,0 Teilen Entschäumer, 0,2 bis 1,5 Teilen Entstaubungsmittel in einer Perlmühle gemahlen (Teile = Gewichtsteile).

Die Farbstoffe eignen sich auch hervorragend zur Herstellung von Mischungen mit anderen Dispersionsfarbstoffen zur Erzeugung z.B. von Braun-, Grau- oder Grüntönen auf der Faser, weil sie die Lichtechtheit dieser Farbstoffe nicht beeinträchtigen.

Die erfindungs gemaβen Farbstoffe (I) können Zusammen mit einem oder mehreren Farbstoffen, wie sie üblicherweise zum Färben von Polyesterfasern oder Polyestertextilmaterialien für Automobilbezugsstoffe verwendet werden eingesetzt werden. Bei diesen Farbstoffen zum Färben von Automobilbezugsstoffen kann es sich insbesondere um Azo-, Disazo-, Anthrachinon-, Nitro-, Naphthalimid- und Terephthalimid-Farbstoffe handeln. Besonders bevorzugte Farbstoffe für derartige Mischungen sind z.B. die Colour-Index-Farbstoffe Yellow 23, 42, 51, 59, 65, 71, 86, 108, 122, 163, 182, 211, Orange 29, 30, 32, 41, 44, 45, 61, 73, Rot 60, 82, 86, 91, 92, 127, 134, 138, 159, 167, 191, 202, 258, 279, 284, 302, 323, Blau 27, 54, 56, 60, 73, 77, 79, 79:1, 87, 266, 333, 361 Violet 27, 28, 57, und 95, wobei sich die Gewichtsverhältnisse der Farbstoffmischungen nach der gewünschten Farbnuance richten.

### Beispiel 1

159,7 g (1,1 Mol) Amino-imino-isoindolenin, 150,2 g (1,0 Mol) 2-Aminobenzothiazol und 750 ml Ethanol werden 24 Stunden unter Rückfluß erhitzt. Nach Abkühlen auf Raumtemperatur wird das ausgefallene Produkt abgesaugt und mit Ethanol und Wasser gewaschen. Nach dem Trocknen bei 70°C erhält man 226,9 g (81,5% d.Th.) eines Produktes der Formel:

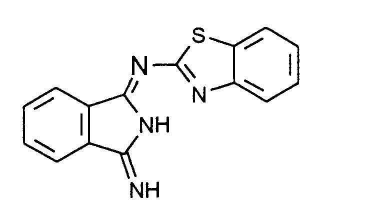

| Analyse: C₁₅H₁₀N₄S (Molgewicht: 278,4) | | | | |
|---|---|---|---|---|
| | C | H | N | S |
| % berechnet | 64,7 | 3,6 | 20,1 | 11,5 |
| % gefunden | 64,3 | 3,5 | 19,9 | 11,8 |

### Beispiel 2

56,4 g (0,44 Mol) Phthalsäuredinitril, 60,1 g (0,4 Mol) 2-Aminobenzthiazol und 300 ml Methanol werden verrührt und anschließend 60 ml einer 30%igen Lösung von Natrium-methylat in Methanol zugefügt. Der Ansatz wird 4 Stunden bei 40°C gerührt, wobei sich bereits ein Niederschlag bildet. Durch Zutropfen von 20,5 ml Eisessig wird die Fällung vervollständigt. Das Produkt wird bei Raumtemperatur abgesaugt und mit Methanol und Wasser gewaschen. Nach dem Trocknen bei 70°C erhält man 102,1 g eines 90,8%igen Produktes der gleichen Struktur wie bei Beispiel 1. Dies entspricht einer Ausbeute von 90,8 % d.Th. Die Substanz ist für die weiteren Umsetzungen ausreichend rein.

### Beispiel 3

Setzt man in Beispiel 1 statt 2-Aminobenzthiazol eine äquivalente Menge 2-Amino-6-methoxy-benzthiazol ein so erhält man in einer Ausbeute von 87,6 % d.Th. ein Produkt der Formel:

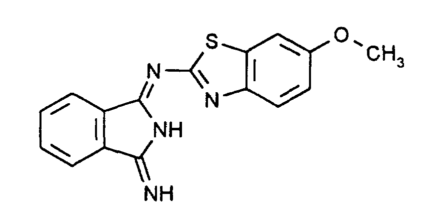

### Beispiel 4

Setzt man in Beispiel 2 statt 2-Aminobenzthiazol eine äquivalente Menge 2-Amino-6-methoxy-benzthiazol ein so erhält man in einer Ausbeute von 91,6 % d.Th. die gleiche Substanz wie in Beispiel 3. Das Produkt ist 97,6%ig.

| Analyse: C₁₆H₁₂N₄OS (Molgewicht: 308,4) | | | |
|---|---|---|---|
| | C | H | S |
| % berechnet | 62,3 | 3,9 | 10,4 |
| % gefunden | 62,2 | 3,7 | 10,6 |

Mit vergleichbarer Ausbeute und Reinheit werden die in der Tabelle 1 aufgeführten Substanzen der Formel X erhalten, wenn man bei der Umsetzung nach den Beispielen 1 bis 4 die analogen substituierten 2-Amino-benzthiazole einsetzt.

**Tabelle 1**

| Beispiel Nr. | R₁ | R₂ | R₃ | R₄ |
|---|---|---|---|---|
| Bsp5 | H | OC₂H₅ | H | H |
| Bsp6 | H | CH₃ | H | H |
| Bsp7 | CH₃ | | | CH₃ |
| Bsp8 | H | H | CH₃ | H |
| Bsp9 | Cl | OCH₃ | | OCH₃ |
| Bsp10 | H | H | -CH=CH-CH=CH- | |

### Beispiel 11

27,8 g (0,1 Mol) einer gemäß Beispiel 1 oder eine äquivalente Menge einer gemäß Beispiel 2 hergestellten Substanz, 400 ml n-Butanol, 17,3 ml (0,12 Mol) Cyanessigsäure-butylester und 6 ml Eisessig werden 6 Stunden auf 90°C erhitzt. Nach Abkühlen auf Raumtemperatur wird die ausgefallenen Substanz abgesaugt und mit Methanol und Wasser gewaschen. Nach dem Trocknen erhält man 36,6 g (90,9 % d.Th.) eines gelben Farbstoffes der folgenden Formel, der Polyesterfasern in grünstichig gelben Tönen mit hervorragender Lichtechtheit färbt.

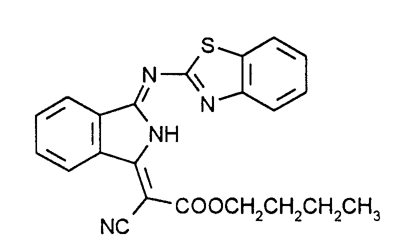

| Analyse: C₂₂H₁₈N₄O₂S (Molgewicht: 402,5) | | | |
|---|---|---|---|
| | C | H | N |
| % berechnet | 65,7 | 4,5 | 13,9 |
| % gefunden | 65,6 | 4,5 | 13,6 |

### Beispiel 12

27,8 g (0,1 Mol) einer gemäß Beispiel 1 oder eine äquivalente Menge einer gemäß Beispiel 2 hergestellten Substanz, 150 ml Wasser, 17,3 ml (0,12 Mol) Cyanessigsäure-butylester und 6 ml Eisessig werden 4 Stunden auf 95°C erhitzt. Nach Abkühlen auf Raumtemperatur wird die ausgefallenen Substanz abgesaugt und mit Methanol und Wasser gewaschen. Nach dem Trocknen erhält man 39,4 g (97,9 % d.Th.) des gleichen gelben Farbstoffes wie bei Beispiel 11.

### Beispiel 13

30,8 g (0,1 Mol) einer gemäß Beispiel 3 oder eine äquivalente Menge einer gemäß Beispiel 4 hergestellten Substanz, 150 ml Wasser, 17,3 ml (0,12 Mol) Cyanessigsäure-amylester, 20 ml n-Amylalkohol und 6 ml Eisessig werden 4 Stunden auf 95°C erhitzt. Nach Abkühlen auf Raumtemperatur wird die ausgefallenen Substanz abgesaugt und mit Methanol und Wasser gewaschen. Nach dem Trocknen erhält man 44,1 g (98,7 % d.Th.) eines gelben Farbstoffes der folgenden Formel, der Polyesterfasern in gelborangen Farbtönen mit ausgezeichneter Lichtechtheit färbt.

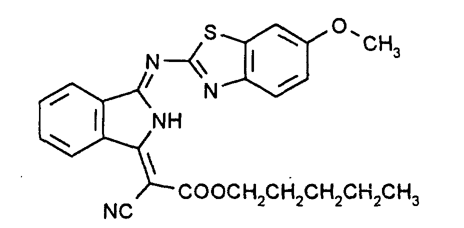

Verfährt man wie in den Beispielen 11, 12 und 13, setzt dabei die in der Tabelle 1 aufgeführten Substanzen als Ausgangsprodukte ein und kondensiert diese mit den entsprechenden Estern der Cyanessigsäure, so erhält man die in der Tabelle 2 angegebenen Farbstoffe der Formel III.

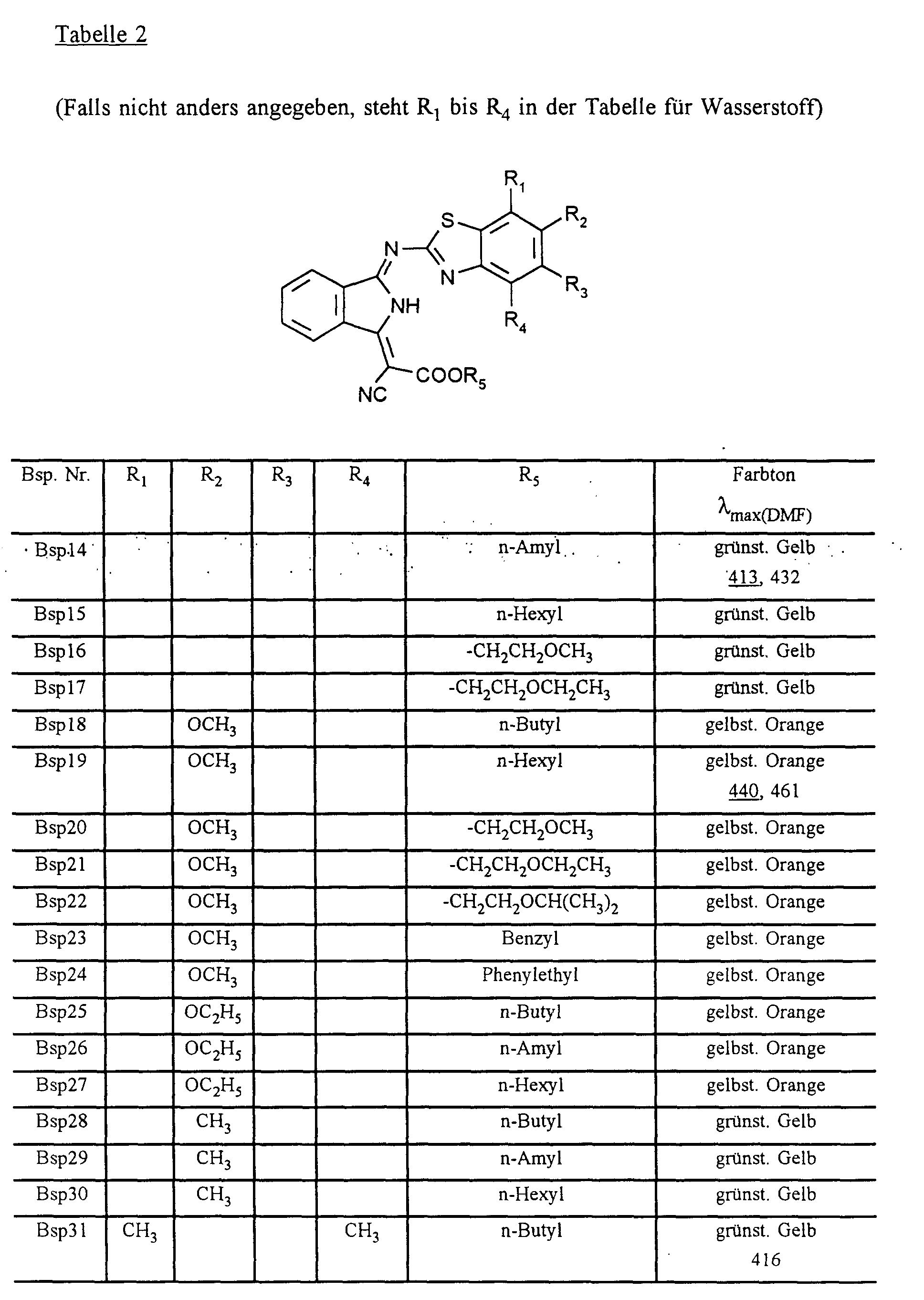

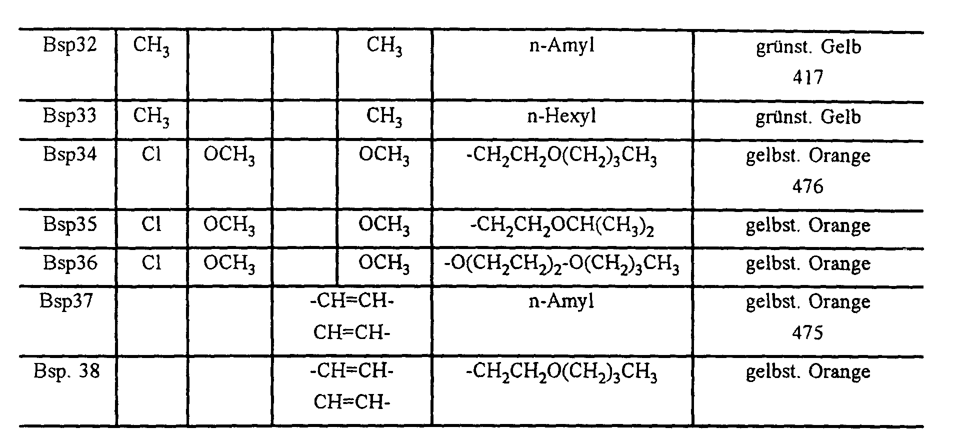

### Beispiel 39

181,5 g (1,25 Mol) Amino-imino-isoindolenin werden in 2500 ml Methanol gelöst und 174,3 g 2-Cyanmethyl-benzthiazol zugegeben. Der Ansatz wird 5 Stunden bei Raumtemperatur und 2 Stunden unter Rückfluß gerührt. Danach wird das ausgefallene Produkt bei Raumtemperatur abgesaugt und mit Methanol gewaschen. Man erhält 290,2 g (96 % d.Th.) einer Substanz der folgenden Formel:

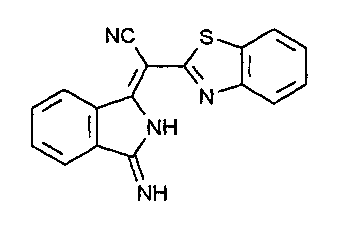

### Beispiel 40

30,5 g (0,21 Mol) Amino-imino-isoindolenin, 34,8 g (0,2 Mol) 2-Cyanmethylbenzthiazol, 200 ml Wasser und 1 g Reax 910, einem Dispergiermittel auf der Basis von Sulfolignin, werden 3 Stunden bei Raumtemperatur, 2 Stunden bei 30°C und 1 Stunde bei 40°C verrührt. Anschließend wird bei 60°C abgesaugt und mit Wasser gewaschen. Man erhält 61,7 g eines Produktes mit gleicher Struktur wie bei Beispiel 39.

### Beispiel 41

28,2 g (0,22 Mol) Phthalsäuredinitril, 34,8 g (0,2 Mol) 2-Cyanmethyl-benzthiazol und 200 ml Methanol werden verrührt und 40 ml einer 30%igen Natriummethylatlösung zugegeben. Die Temperatur steigt innerhalb von 30 Minuten auf 40°C, und es bildet sich ein dunkler Niederschlag. Nach 5 Stunden säuert man mit 15 ml Eisessig an, saugt ab und wäscht mit Methanol und Wasser. Man erhält 55,3 g eines Produktes mit gleicher Struktur wie bei Beispiel 39 mit einer Reinheit von ca. 94 %.

### Beispiel 42

Verwendet man an Stelle von Natriummethylat 22 ml einer 10 N Natronlauge, verfährt aber sonst wie bei Beispiel 41, so erhält man 55,9 g eines Produktes mit gleicher Struktur wie bei Beispiel 39 mit einer Reinheit von ca. 93,6 %.

Mit vergleichbarer Ausbeute und Reinheit werden die in der Tabelle 3 aufgeführten Substanzen der Formel IX erhalten, wenn man bei der Umsetzung wie bei den Beispielen 39 bis 42 verfährt, aber die analogen, substituierten 2-Cyanmethylbenzthiazole einsetzt.

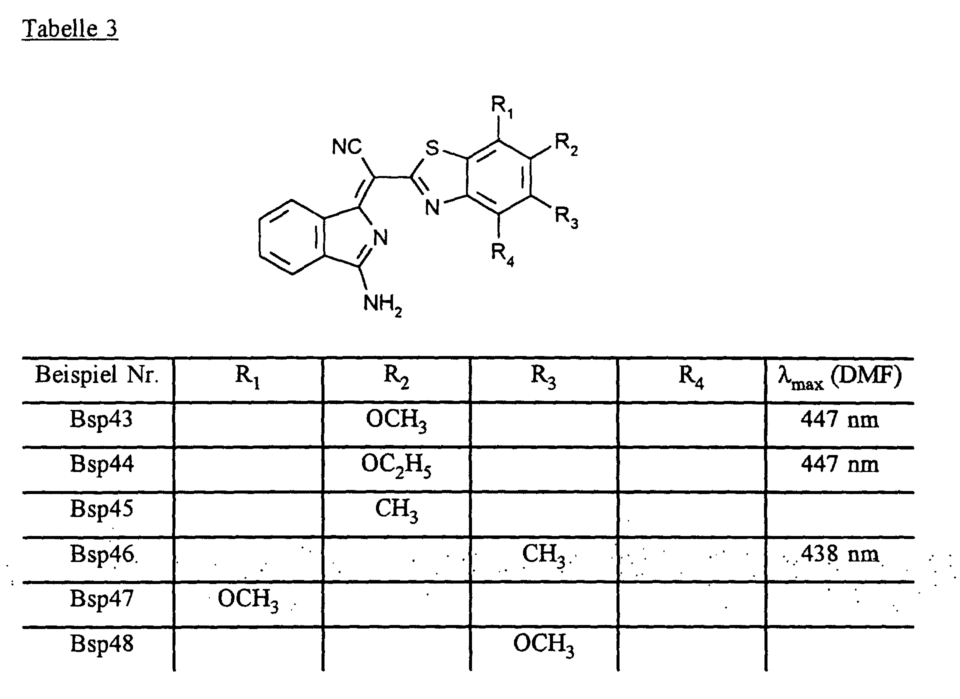

### Beispiel 49

60,5 g (0,2 Mol) einer gemäß Beispiel 39 oder eine äquivalente Menge einer nach den Beispielen 40 bis 42 hergestellten Substanz, 4,8 g Reax 910, 200 ml Wasser und 12 ml Eisessig werden verrührt. Hierzu gibt man 42,6 g (0,23 Mol) Cyanessigsäure-(2-butoxy-ethyl)-ester und 30 ml 2-Butoxy-ethanol und erhitzt 3 Stunden auf 80°C und 2 Stunden auf 95°C Danach wird das Produkt abgesaugt und mit Wasser gewaschen. Man erhält 89,8 g einer nach HPLC 88%igen Substanz der folgenden Formel mit einem λₘₐₓ (DMF + 5% Eisessig) von 458 nm. Der Farbstoff färbt Polyestermaterialien in gelben, fluoreszierenden Farbtönen von sehr hoher Lichtechtheit.

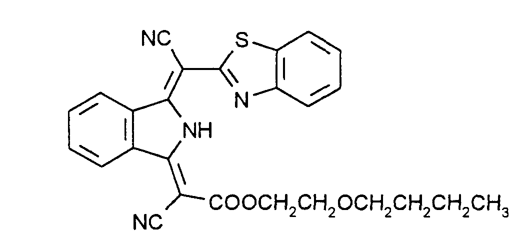

### Beispiel 50

42,6 g (0,23 Mol) Cyanessigsäure-(2-butoxy-ethyl)-ester, 60,5 g (0,2 Mol) einer gemäß Beispiel 39 oder eine äquivalente Menge einer nach den Beispielen 40 bis 42 hergestellten Substanz, 12 ml Eisessig und 500 ml 2-Butoxy-ethanol werden 3 Stunden auf 80°C erhitzt. Man erhält 84,8 g einer Substanz der gleichen Struktur wie bei Beispiel 49.

### Beispiel 51

30,5 g (0,21 Mol) Amino-imino-isoindolenin, 34,8 g (0,2 Mol) 2-Cyanmethylbenzthiazol, 2 g Reax 910 und 200 ml Wasser werden 3 Stunden bei 30°C und 1 Stunde bei 40°C verrührt. Nunmehr werden weitere 2 g Reax 910, 24 ml Eisessig, 44,4 g (0,24 Mol) Cyanessigsäure-(2-butoxy-ethyl)-ester und 30 ml 2-Butoxyethanol zugegeben und 2 Stunden auf 80°C und 2 Stunden auf 90°C erhitzt. Danach wird abgesaugt und mit Wasser gewaschen. Man erhält 94,8 einer Substanz der gleichen Struktur wie bei Beispiel 50 mit einer Reinheit von 71 % nach HPLC.

Verfährt man wie in den Beispielen 49 und 50, setzt dabei die in der Tabelle 3 aufgeführten Substanzen als Ausgangsprodukte ein und kondensiert diese mit den entsprechenden Estern der Cyanessigsäure, so erhält man die in der Tabelle 4 angegebene Farbstoffe der Formel II.

**Tabelle 4**

| (Falls nicht anders angegeben, steht R₁ bis R₄ in der Tabelle für Wasserstoff) | | | | | | |
|---|---|---|---|---|---|---|
| Bsp. Nr. | R₁ | R₂ | R₃ | R₄ | R₅ | Farbton/ λₘₐₓ |
| Bsp52 | | | | | n-Butyl | |
| Bsp53 | | | | | n-Amyl | |
| Bsp54 | | | | | n-Hexyl | |
| Bsp55 | | | | | -CH₂CH₂OCH(CH₃)₂ | |
| Bsp56 | | OCH₃ | | | n-Hexyl | rotstichig Orange |
| Bsp57 | | OCH₃ | | | -CH₂CH₂O(CH₂)₃CH₃ | rotstichig Orange 467,499nm |
| Bsp58 | | CH₃ | | | -CH₂CH₂O(CH₂)₃CH₃ | Gelb 467,499nm |
| Bsp59 | | | CH₃ | | -CH₂CH₂O(CH₂)₃CH₃ | Gelb 462 nm |
| Bsp60 | | OC₂H₅ | | | n-Amyl | rotstichig Orange |
| Bsp61 | | OC₂H₅ | | | n-Hexyl | rotstichig Orange 478 nm |
| Bsp62 | | OC₂H₅ | | | -CH₂CH₂O(CH₂)₃CH₃ | rotstichig Orange |

### Beispiel 63

34,6 g der Substanz von Beispiel 44, 11 ml n-Butylamin, 6 ml Eisessig und 250 ml n-Butanol werden 2 Stunden auf 80°C und 3 Stunden unter Rückfluß erhitzt. Das Produkt wird bei Raumtemperatur abgesaugt und aus DME/Wasser umkristallisiert. Man erhält 28 g eines Produktes der folgenden Formel, das Polyesterfasern in grünstichig gelben Tönen färbt. Das Produkt besitzt in DMF ein Absorptionsmaximum bei 448 nm.

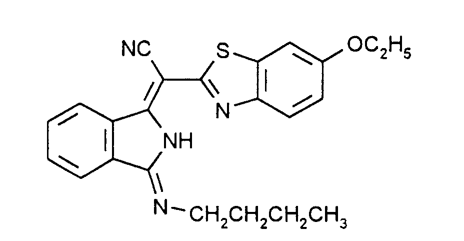

Analog zu Beispiel 63 können die in der Tabelle 5 aufgeführten Verbindungen der allgemeinen Formel (IV) hergestellt werden.

**Tabelle 5**

| (Falls nicht anders angegeben, steht R₁ bis R₄ in der Tabelle für Wasserstoff) | | | | | | |
|---|---|---|---|---|---|---|
| Bsp.Nr. | R₁ | R₂ | R₃ | R₄ | R₅ | Farbton λₘₐₓ (DMF) |
| 64 | | CH₃ | | | n-Hexyl | grünst. Gelb 443 nm |
| 65 | | | CH₃ | | n-Butyl | grünst. Gelb 438 nm |
| 66 | | OCH₃ | | | n-Hexyl | grünst. Gelb 450 nm |
| 67 | | OC₂H₅ | | | n-Butyl | grünst. Gelb 448 nm |
| 68 | | OC₂H₅ | | | n-Hexyl | grünst. Gelb 451 nm |
| 69 | | OC₂H₅ | | | -CH₂CH₂OCH₃ | grünst. Gelb |

### Beispiel 70

70a) 159,7 g (1,1 Mol) Amino-imino-isoindolenin, 150,2 g (1,0 Mol) 2-Aminobenzothiazol und 750 ml Ethanol werden 24 Stunden unter Rückfluß erhitzt. Nach Abkühlen auf Raumtemperatur wird das ausgefallene Produkt abgesaugt und mit Ethanol und Wasser gewaschen. Nach dem Trocknen bei 70°C erhält man 226,9 g (81,5 % der Theorie) eines Produktes der Formel:

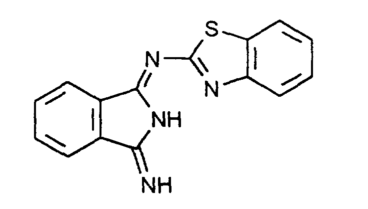

| Analyse: C₁₅H₁₀N₄S (Molgewicht: 278,4) | | | | |
|---|---|---|---|---|
| | C | H | N | S |
| % berechnet | 64,7 | 3,6 | 20,1 | 11,5 |
| % gefunden | 64,3 | 3,5 | 19,9 | 11,8 |

70b) 27,8 g (0,1 Mol) einer gemäß Beispiel 70a hergestellten Substanz, 400 ml n-Butanol, 17,3 ml (0,12 Mol) Cyanessigsäure-butylester und 6 ml Eisessig werden 6 Stunden auf 90°C erhitzt. Nach Abkühlen auf Raumtemperatur wird die ausgefallene Substanz abgesaugt und mit Methanol und Wasser gewaschen. Nach dem Trocknen erhält man 36,6 g (90,9 % der Theorie) eines gelben Farbstoffes der Formel

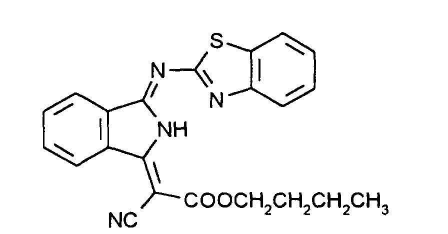

70c) Man verfährt wie in Beispiel 70b, ersetzt jedoch den Cyanessigsäure-butylester durch eine äquivalente Menge Cyanessigsäure-(2-butoxy-ethyl)-ester und n-Butanol durch das gleiche Volumen an 2-Butoxy-ethanol, so erhält man in vergleichbarer Ausbeute den Farbstoff der Formel

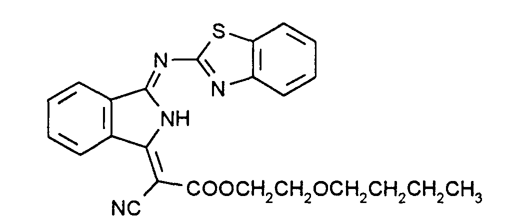

70d) 27,8 g (0,1 Mol) einer gemäß Beispiel 70a hergestellten Substanz, 150 ml Wasser, 18,3 g (0,12 Mol) einer Mischung aus 75 % Cyanessigsäure-butylester und 25 % Cyanessigsäure-(2-butoxy-ethyl)-ester und 6 ml Eisessig werden 4 Stunden auf 95°C erhitzt. Nach Abkühlen auf Raumtemperatur wird die ausgefallene Substanz abgesaugt und mit Methanol und Wasser gewaschen. Nach dem Trocknen erhält man 39,4 g einer Mischung der beiden Farbstoffe der Beispiele 70b und 70c.

### Formierungen

70e) 30 g des in Beispiel 70b erhaltenen Farbstoffs (in Form des wasserfeuchten Preßkuchens) werden in 200 ml Wasser mit 63 g Natriumligninsulfonat und 3 g eines nichtionogenen Dispergiermittels (Additionsprodukt aus Abietinsäure und 50 Mol-Äquivalente Ethylenoxid) versetzt und mit 25 %iger Schwefelsäure auf einen pH-Wert von 7 gestellt. Anschließend wird 1 Stunde bei Raumtemperatur in der Perlmühle bis zur Feinverteilung (90 % ≤ 1 µm) gemahlen, gesiebt und im Sprühtrockner getrocknet.

70f) Man verfährt wie in Beispiel 70e), ersetzt jedoch den Farbstoff 70b durch den Farbstoff 70c.

70g) Man verfährt wie im Beispiel 70e, ersetzt jedoch 8,0 g der 30 g vom Farbstoff 70b durch den Farbstoff aus Beispiel 70c und erhält eine Misch-Formierung.

70h) 25,5 bis 21,0 g Farbstoff aus Beispiel 70b und 4,5 bis 9,0 g Farbstoff aus Beispiel 70c werden in 200 ml Wasser mit 63 g Natriumligninsulfonat und 1,5 bis 6 g des nichtionogenen Dispergiermittels aus Beispiel 70e versetzt. Anschließend wird 1 Stunde in einer Perlmühle bis zur Feinverteilung (90 % ≤ 1 µm) gemahlen, wobei die Temperatur für ca. 15 Minuten auf 80 bis 90°C angehoben wurde. Die jeweiligen Farbstoffmischungen werden gesiebt und sprühgetrocknet.

70i) Anstelle der beiden Farbstoffe aus Beispiel 70b und 70c werden 30 g Mischfarbstoff aus Beispiel 70d eingesetzt. Ansonsten verläuft der Formierprozeß analog dem Beispiel 70e.

### Anwendungsbeispiel 1

2 g des nach Beispiel 70e erhaltenen Pulvers werden in 1 000 g Wasser dispergiert. Die Dispersion wird mit 0,5 bis 2 g/l eines handelsüblichen Dispergiermittels auf Basis eines Kondensationsproduktes aus Naphthalinsulfonsäurenatriumsalz und Formaldehyd, 0,5 bis 2 g/l Mononatriumphosphat und 2 g eines handelsüblichen Egalisierhilfsmittels versetzt und mit Essigsäure auf einen pH-Wert von 4,5 bis 5,5 gestellt. In die so erhaltene Färbeflotte bringt man 100 g eines texturierten Polyestergewebes auf Basis Polyethylenglykolterephthalat ein und färbt 60 Minuten bei 130°C.

In analoger Weise wurden auch die nach den Beispielen 70f bis 70i erhaltenen Farbstoffpräparate zum Färben verwendet. Insbesondere war während der Aufheizphase zwischen 90 und 100°C eine deutlich verbesserte Färbebadstabilität bei den Farbstoffmischungen gegenüber den Einzelfarbstoffen zu erkennen.

### Beispiel 71

71a) 181,5 g (1,25 Mol) Amino-imino-isoindolenin werden in 2500 ml Methanol gelöst und 174,3 g 2-Cyanmethyl-benzthiazol zugegeben. Der Ansatz wird 5 Stunden bei Raumtemperatur und 2 Stunden unter Rückfluß gerührt. Danach wird das ausgefallene Produkt bei Raumtemperatur abgesaugt und mit Methanol gewaschen. Man erhält 290,2 g (96 % der Theorie) einer Substanz der folgenden Formel

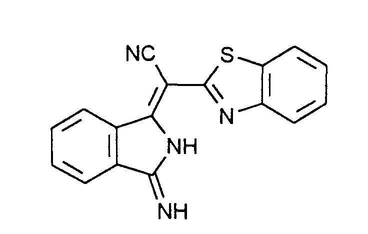

71b) 60,5 g (0,2 Mol) einer gemäß Beispiel 71a) hergestellten Substanz 4,8 g Reax® 910, 200 ml Wasser und 12 ml Eisessig werden verrührt. Hierzu gibt man 42,6 g (0,23 Mol) Cyanessigsäure-(2-butoxy-ethyl)-ester und 30 ml 2-Butoxyethanol und erhitzt 3 Stunden auf 80°C und 2 Stunden auf 95°C. Danach wird das Produkt abgesaugt und mit Wasser gewaschen. Man erhält 89,8 g einer nach HPLC 88 %igen Substanz der folgenden Formel mit einem λₘₐₓ (DMF + 5 % Eisessig) von 458 nm. Der Farbstoff färbt Polyestermaterialien in gelben, fluoreszierenden Farbtönen von sehr hoher Lichtechtheit.

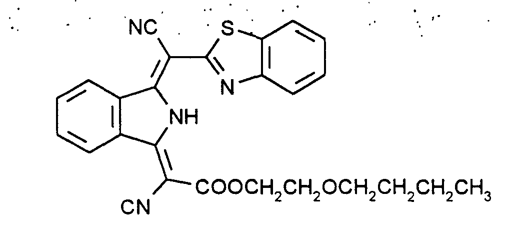

71c) Man verfährt wie bei Beispiel 71b, ersetzt jedoch den Cyanessigsäure-(2-butoxy-ethyl)-ester durch eine entsprechende Menge des Amylesters, so erhält man in vergleichbarer Ausbeute den Farbstoff der Formel

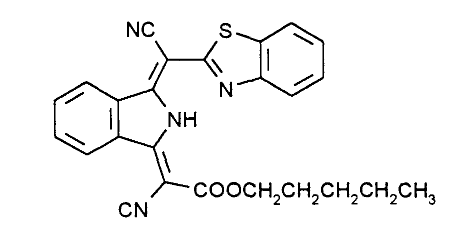

71d) Man verfährt wie bei Beispiel 71b, ersetzt jedoch den Cyanessigsäure-(2-butoxy-ethyl)-ester durch eine äquivalente Mischung aus 75 % des Cyanessigsäure-(2-butoxy-ethyl)-esters und 25 % des Amylesters, so erhält man in vergleichbarer Ausbeute unmittelbar eine Mischung der beiden Farbstoffe aus den Beispielen 71b und 71d.

### Anwendungsbeispiel 2

Verfährt man wie bei dem Anwendungsbeispiel 1, ersetzt jedoch die Farbstoffe durch die analogen Farbstoffe aus den Beispielen 70 und 71, so erhält man ebenfalls farbstarke brillante Gelbfärbungen mit hervorragenden coloristischen Eigenschaften, wobei die thermische Stabilität der Farbstoffdispersionen im Färbebad ebenfalls gegenüber den entsprechenden Dispersionen der Einzelfarbstoffe erheblich verbessert ist.

## Patentansprüche

1. Farbstoffe der Formel (I) 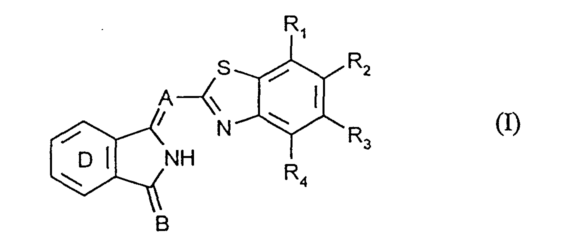 worin
A für N oder einen Cyanmethylenrest,
B für einen Rest der Formel C(CN)(COOR₅ oder N-R₆ steht; mit der Maβgabe, dass A für einen Cyanmethylenrest steht, falls B für N-R₆ steht,
R₁ bis R₄ unabhängig voneinander Wasserstoff, Halogen, gegebenenfalls substituiertes C₁-C₈-Alkyl oder C₅-C₈-Cycloalkyl, gegebenenfalls durch Sauerstoff unterbrochenes C₁-C₁₀-Alkoxy, gegebenenfalls substituiertes C₆-C₁₀-Aryloxy, CF₃, oder gegebenenfalls substituiertes Dialkylamin bedeuten oder jeweils zwei benachbarte R₁ bis R₄-Reste zusammen mit den aromatischen Ring C-Atomen einen annelierten Benzol- oder Naphthalinring bilden, der gegebenenfalls weiter substituiert ist,
R₅ für einen gegebenenfalls substituierten und gegebenenfalls durch Sauerstoff unterbrochenen, gesättigten oder ungesättigten C₁-C₂₀-Alkylrest, C₆-C₁₀₋Aryl-C₁-C₁₀-alkyl oder Hetarylalkyl steht,
R₆ gegebenenfalls substituiertes und gegebenenfalls durch Sauerstoff unterbrochenes C₁-C₂₀-Alkyl, Cycloalkyl, Cycloalkyl-alkyl, oder Aralkyl bedeutet und
der Ring D unsubstituiert ist oder wenigstens einen Substituenten trägt, welcher gegebenenfalls, zusammen mit einem weiteren Substituenten in o-Stellung und den Ring-C-Atomen, einen annelierten Benzol- oder Naphthalinring bildet, mit Ausnahme des Farbstoffs der Formel

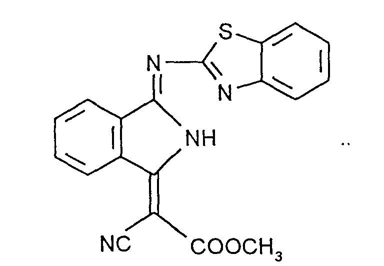

2. Farbstoffe der Formel (I) gemäß Anspruch 1, worin
R₁ und R₂ unabhängig voneinander Wasserstoff, Cl, Br, Methyl, Ethyl, n-Propyl, Isopropyl, n-Butyl, iso-Butyl, tert.-Butyl, Cyclohexyl, gegebenenfalls durch 1 bis 2 Sauerstoffe unterbrochenes C₁-C₁₀-Alkoxy, gegebenenfalls substituiertes Phenoxy, CF₃ oder eine Di(C₁-C₄)-Alkylaminogruppe bedeuten,
R₃ und R₄ die Bedeutung von R₁ und R₂ haben oder zusammen mit den Ring-C-Atomen einen annelierten Benzolring bilden,
R₅ einen gegebenenfalls durch Cl, CN oder gegebenenfalls substituiertes Phenoxy, substituiertes und gegebenenfalls durch 1 bis 2 Sauerstoffatome unterbrochenes C₁-C₁₂-Alkyl, C₆-C₁₀-Aryl-C₁-C₁₀-Alkyl oder Hetarylalkyl,
R₆ einen gegebenenfalls durch gegebenenfalls substituiertes Phenoxy, substituiertes gesättigtes oder ungesättigtes C₁-C₁₂-Alkyl, das gegebenenfalls durch 1 bis 2 Sauerstoffe unterbrochen ist und
Ring D unsubstituiert oder durch CN, Halogenatome, insbesondere 1 bis 4 Cl-Atomen, 1 bis 2 C₁-C₁₀-Alkylreste und/oder 1 bis 2 C₁-C₁₀-Alkoxyreste oder einen Phenylrest substituiert ist, die gegebenenfalls jeweils durch 1 bis 2 Sauerstoffatome unterbrochen sind.

3. Farbstoffe gemäß Anspruch 1 der Formel (II) 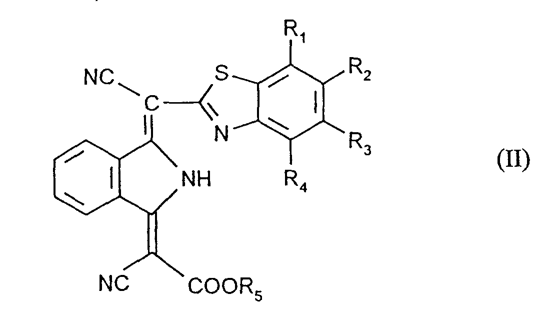 worin R₁ bis R₅ die Bedeutungen gemäß Anspruch 1 haben.

4. Farbstoffe gemäß Anspruch 1 der Formel (III) 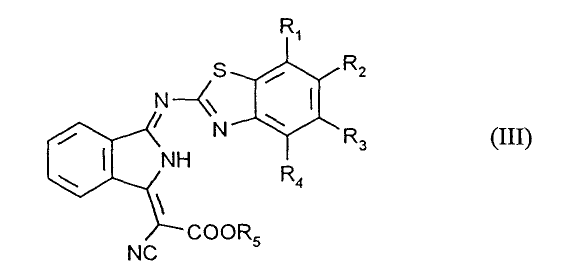 worin R₁ bis R₅ die Bedeutungen gemäß Anspruch 1 besitzen.

5. Farbstoffe gemäß Anspruch 1 der Formel (IV) 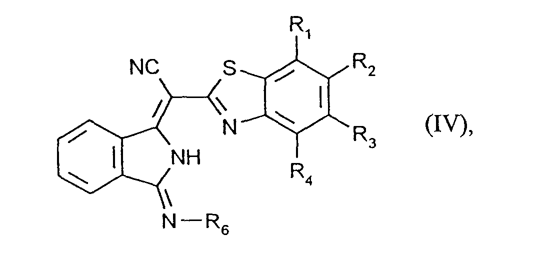 worin R₁ bis R₄ und R₆ die in Anspruch 1 angegebenen Bedeutungen haben.

6. Verfahren zur Herstellung von Verbindungen der Formel (II) gemäß Anspruch 3, **dadurch gekennzeichnet, daß** man ein Aminoisoindolenin der Formel (VII) 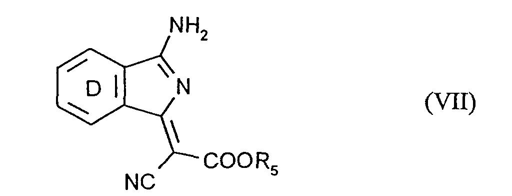 mit einem 2-Cyanmethyl-benzthiazol der Formel (VIII) 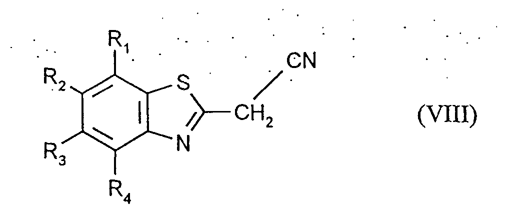 oder ein Aminoisoindolenin der Formel (IX) 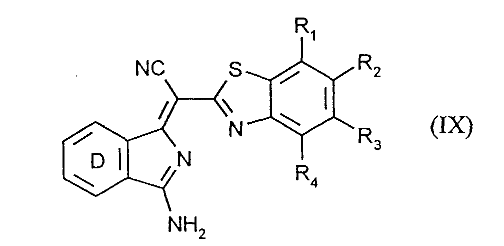 mit einem Cyanessigsäureester der Formel (VI)
NC-CH₂-COOR₅ (VI),
kondensiert, worin R₁ bis R₅ und D die gemäß Anspruch 3 angegebenen Bedeutungen besitzen.

7. Verfahren zur Herstellung von Farbstoffen der Formel (III) gemäß Anspruch 4, **dadurch gekennzeichnet, daß** Verbindungen der Formel (X) 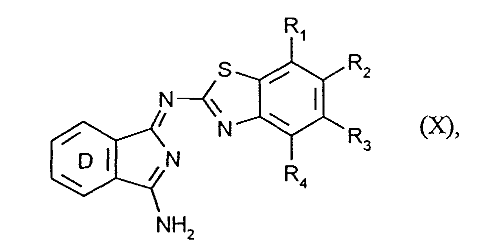 worin D und R₁ bis R₄ die Bedeutung gemäß Anspruch 4 haben,
mit Cyanessigsäureestern der Formel (VI)
NC-CH₂-COOR₅ (VI),
worin R₅ die Bedeutung gemäß Anspruch 4 hat, kondensiert werden.

8. Verfahren zur Herstellung von Verbindungen der Formel (IV) gemäß Anspruch 5, **dadurch gekennzeichnet, daß** Aminoisoindolenine der Formel (IX) 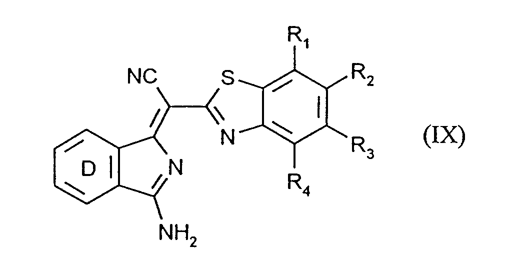 mit Aminen der Formel R₆-NH₂ umgesetzt werden, wobei D, R₁ bis R₄ und R₆ die Bedeutungen gemäß Anspruch 5 haben.

9. Verfahren zur Herstellung von Verbindungen der Formel (IV) gemäß Anspruch 5, **dadurch gekennzeichnet, daß** man Verbindungen der Formel (XIII) 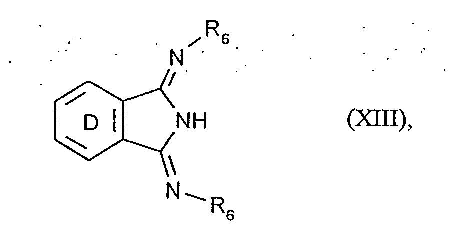 worin R₆ und D die obige Bedeutung besitzen,
mit Verbindungen der Formel (VIII) 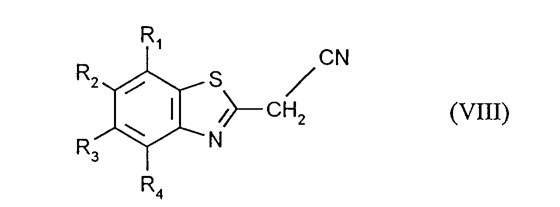 worin R₁ bis R₄ die Bedeutung gemäß Anspruch 5 besitzen, umsetzt.

10. Verwendung von Farbstoffen der Formel (I) gemäß Anspruch 1 zum Farben von vollsynthetischen oder halbsynthetischen, hochmolekularen Stoffen.

## Claims

1. Dyestuffs of the formula (I) 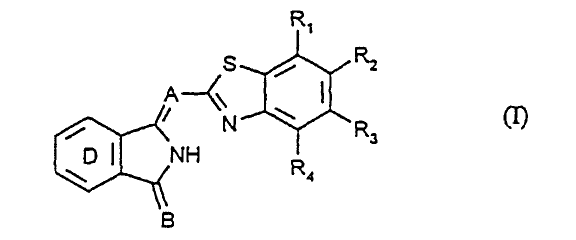 in which
A represents N or a cyanomethylene radical,
B represents a radical of the formula C(CN)COOR₅ or N-R₆, with the proviso that A represents a cyanomethylene radical when B represents N-R₆,
R₁ to R₄, independently of one another, denote hydrogen, halogen, substituted or unsubstituted C₁-C₈-alkyl or C₅-C₈-cycloalkyl, uninterrupted or oxygen-interrupted C₁-C₁₀-alkoxy, substituted or unsubstituted C₆-C₁₀-aryloxy, CF₃, or substituted or unsubstituted dialkylamine, or any two adjacent R₁ to R₄ radicals together with the aromatic ring C atoms form a fused benzene or naphthalene ring which, if desired, is further substituted,
R₅ represents a substituted or unsubstituted, uninterrupted or oxygen-interrupted, saturated or unsaturated C₁-C₂₀-alkyl radical, C₆-C₁₀-aryl-C₁-C₁₀-alkyl or hetarylalkyl
R₆ denotes substituted or unsubstituted, uninterrupted or oxygen-interrupted C₁-C₂₀-alkyl, cycloalkyl, cycloalkyl-alkyl, or aralkyl, and
ring D is unsubstituted or carries at least one substituent which, if desired, together with a further substituent in the o position and the ring C atoms forms a fused benzene or naphthalene ring, with exception of the dyestuff of the formula

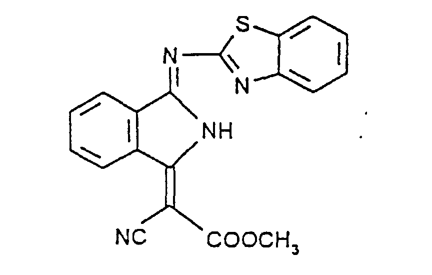

2. Dyestuffs of the formula (I) according to Claim 1, in which
R₁ and R₂, independently of one another, denote hydrogen, Cl, Br, methyl, ethyl, n-propyl, isopropyl, n-butyl, iso-butyl, tert-butyl, cyclohexyl, C₁-C₁₀-alkoxy which may be interrupted by 1 to 2 oxygens, substituted or unsubstituted phenoxy, CF₃ or a di(C₁-C₄)-alkylamino group,
R₃ and R₄ have the meaning of R₁ and R₂ or together with the ring C atoms form a fused benzene ring,
R₅ denotes C₁-C₁₂-alkyl, C₆-C₁₀-aryl-C₁-C₁₀-alkyl or hetarylalkyl which is unsubstituted or substituted by Cl, CN or substituted or unsubstituted phenoxy and may be interrupted by 1 to 2 oxygen atoms,
R₆ denotes saturated or unsaturated C₁-C₁₂-alkyl which is unsubstituted or substituted by substituted or unsubstituted phenoxy and may be interrupted by 1 to 2 oxygens, and
ring D is unsubstituted or substituted by CN, halogen atoms, in particular 1 to 4 Cl atoms, 1 to 2 C₁-C₁₀-alkyl radicals and/or 1 to 2 C₁-C₁₀-alkoxy radicals, or a phenyl radical, each of which is uninterrupted or interrupted by 1 to 2 oxygen atoms.

3. Dyestuffs according to Claim 1 of the formula (II) 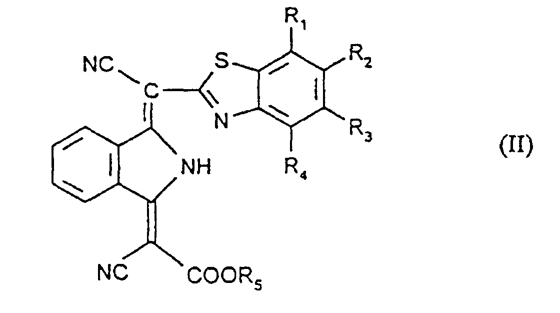 in which R₁ to R₅ have the meanings according to Claim 1.

4. Dyestuffs according to Claim 1 of the formula (III) 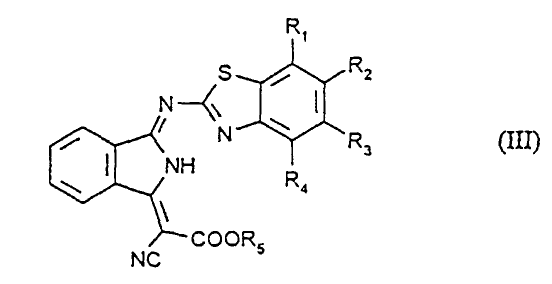 in which R₁ to R₅ have the meanings according to Claim 1.

5. Dyestuffs according to Claim 1 of the formula (IV) 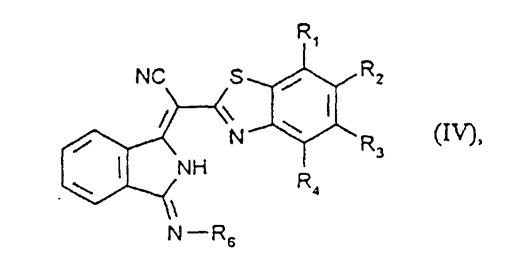 in which R₁ to R₄ and R₆ have the meanings given in Claim 1.

6. Process for preparing compounds of the formula (II) according to Claim 3, **characterized in that** an aminoisoindolenine of the formula (VII) 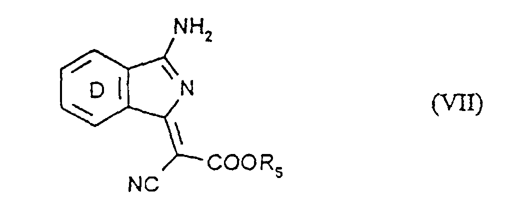 is condensed with a 2-cyanomethyl-benzothiazole of the formula (VIII) 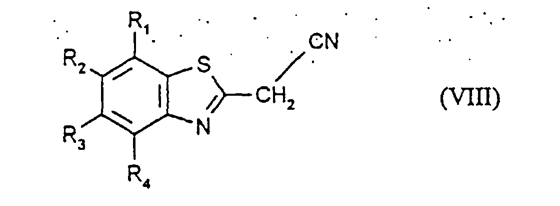 or an aminoisoindolenine of the formula (IX) 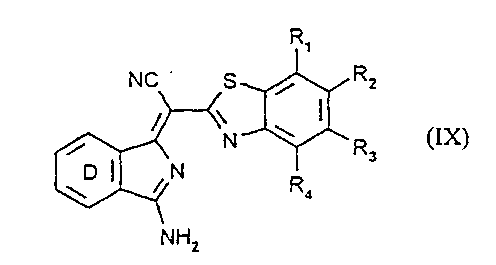 is condensed with a cyanoacetic ester of the formula (VI)
NC-CH₂-COOR₅ (VI),
in which R₁ to R₅ and D have the meanings given in Claim 3.

7. Process for preparing dyestuffs of the formula (III) according to Claim 4, **characterized in that** compounds of the formula (X) 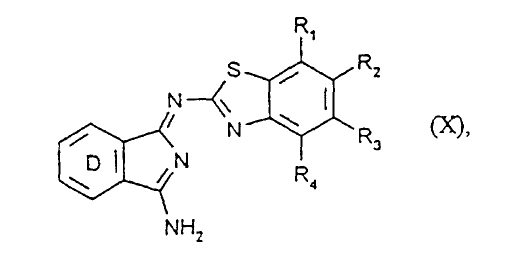 in which D and R₁ to R₄ have the meaning according to Claim 4 are condensed with cyanoacetic esters of the formula (VI)
NC-CH₂-COOR₅ (VI),
in which R₅ has the meaning according to Claim 4.

8. Process for preparing compounds of the formula (IV) according to Claim 5, **characterized in that** aminoisoindolenines of the formula (IX) 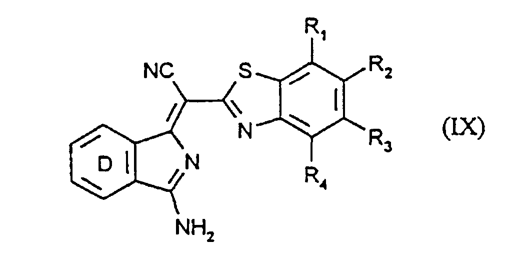 are reacted with amines of the formula R₆-NH₂ where D, R₁ to R₄ and R₆ have the meanings according to Claim 5.

9. Process for preparing compounds of the formula (IV) according to Claim 5, **characterized in that** compounds of the formula (XIII) 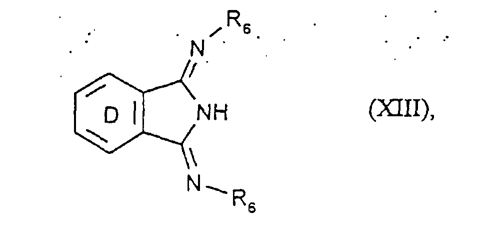 in which R₆ and D have the above meaning are reacted with compounds of the formula (VIII) 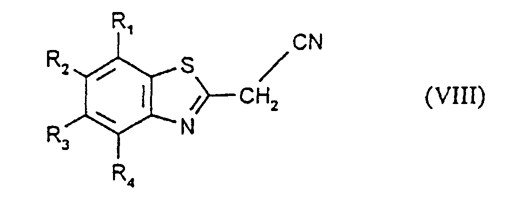 in which R₁ to R₄ have the meaning according to Claim 5.

10. Use of dyestuffs of the formula (I) according to Claim 1 for dyeing fully synthetic or semisynthetic high-molecular-weight materials.

## Revendications

1. Colorants de formule (I) 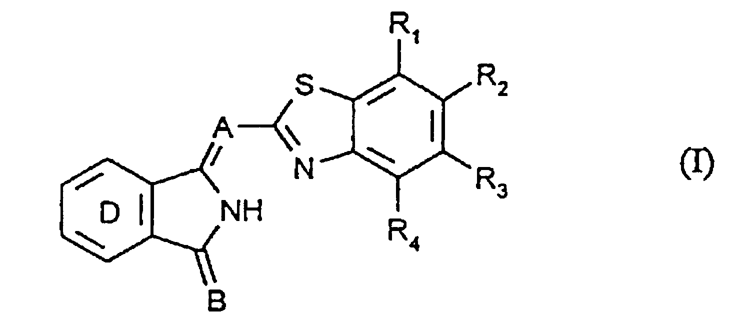 dans laquelle
A représente N ou le groupe cyanométhylène,
B représente un radical de formule C(CN)COOR₅ ou N-R₆, étant entendu que A représente le groupe cyanométhylène lorsque B représente N-R₆,
R₁ à R₄ représentent, indépendamment l'un de l'autre, un atome d'hydrogène ou d'halogène, un groupe alkyle en C₁-C₈ ou cycloalkyle en C₅-C₈ éventuellement substitué, alcoxy en C₁-C₁₀ éventuellement interrompu par des atomes d'oxygène, aryloxy en C₆-C₁₀ éventuellement substitué, CF₃ ou un groupe dialkylamino éventuellement substitué, ou deux radicaux R₁ à R₄ contigus forment ensemble, conjointement avec les atomes de carbone du cycle aromatique, un cycle benzénique ou naphtalénique qui est éventuellement substitué davantage,
R₅ représente un radical alkyle en C₁-C₂₀ saturé ou insaturé, éventuellement substitué et éventuellement interrompu par des atomes d'oxygène, ou un groupe aryl(C₆-C₁₀)-alkyle(C₁-C₁₀) ou hétéroarylalkyle,
R₆ représente un groupe alkyle en C₁-C₂₀, cycloalkyle; cycloalkylalkyle ou aralkyle éventuellement substitué et éventuellement interrompu par des atomes d'oxygène, et
le cycle D est non substitué ou porte au moins un substituant qui éventuellement forme, conjointement avec un autre substituant en position ortho et les atomes de carbone formant le cycle, un cycle benzénique ou naphtalénique soudé, à l'exclusion du colorant de formule

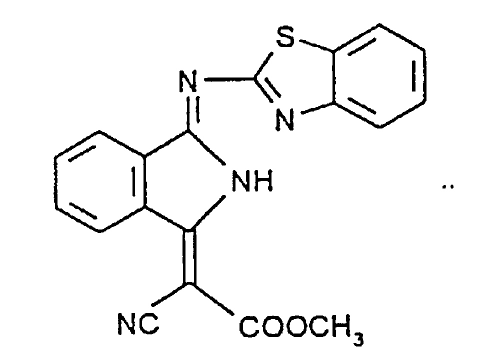

2. Colorants de formule (I) selon la revendication 1, dans lesquels
R₁ et R₂ représentent, indépendamment l'un de l'autre, un atome d'hydrogène ou de Cl, Br, un groupe méthyle, éthyle, n-propyle, isopropyle, n-butyle, isobutyle, tert-butyle, cyclohexyle, un groupe alcoxy en C₁-C₁₀ éventuellement interrompu par 1 ou 2 atomes d'oxygène, un groupe phénoxy éventuellement substitué, CF₃ ou un groupe dialkyl(C₁-C₄)amino,
R₃ et R₄ ont les significations de R₁ et R₂ ou forment ensemble, avec les atomes de carbone formant le cycle, un cycle benzénique soudé,
R₅ représente un groupe alkyle en C₁-C₁₂ éventuellement substitué par Cl, CN ou par un groupe phénoxy éventuellement substitué, et éventuellement interrompu par 1 ou 2 atomes d'oxygène, ou un groupe aryl(C₆-C₁₀)-alkyle(C₁-C₁₀) ou hétéroarylalkyle,
R₆ représente un groupe alkyle en C₁-C₁₂, saturé ou insaturé, éventuellement substitué par un groupe phénoxy éventuellement substitué, qui est éventuellement interrompu par 1 ou 2 atomes d'oxygène, et
le cycle D est non substitué ou substitué par CN, des atomes d'halogène, en particulier 1 à 4 atomes de Cl, ou par 1 ou 2 radicaux alkyle en C₁-C₁₀ et/ou 1 ou 2 radicaux alcoxy en C₁-C₁₀ ou un radical phényle, qui sont éventuellement interrompus chacun par 1 ou 2 atomes d'oxygène.

3. Colorants selon la revendication 1, de formule (II) 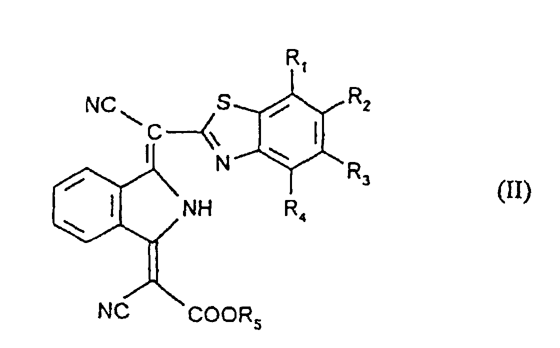 dans laquelle R₁ à R₅ ont les significations indiquées dans la revendication 1.

4. Colorants selon la revendication 1, de formule (III) 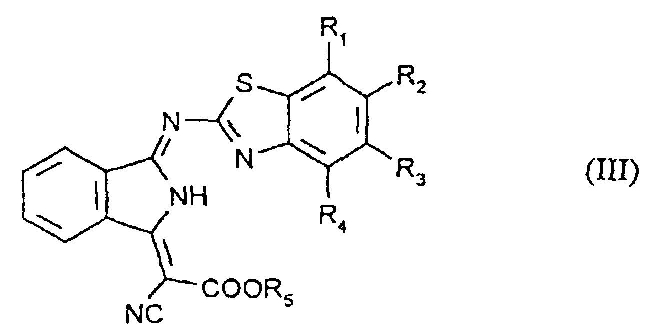 dans laquelle R₁ à R₅ ont les significations selon la revendication 1.

5. Colorants selon la revendication 1, de formule (IV) 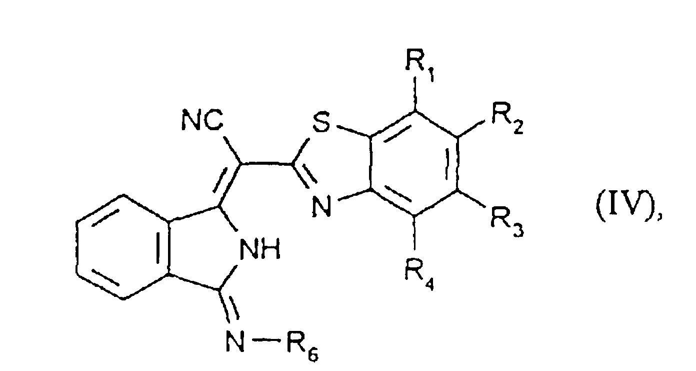 dans laquelle R₁ à R₄ et R₆ ont les significations selon la revendication 1.

6. Procédé pour la préparation des composés de formule (II) selon la revendication 3, **caractérisé en ce qu'**on condense une amino-iso-indolénine de formule (VII) 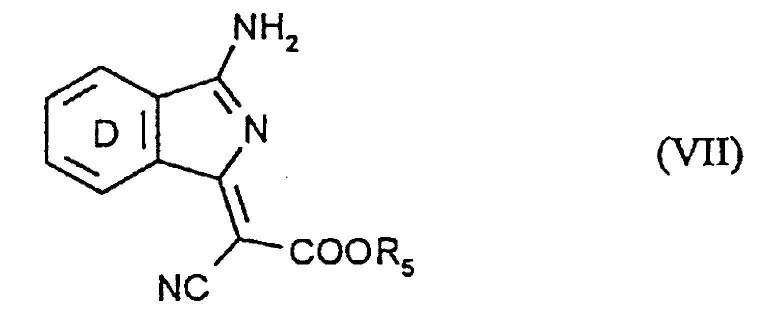 avec un 2-cyanométhylbenzothiazole de formule (VIII) 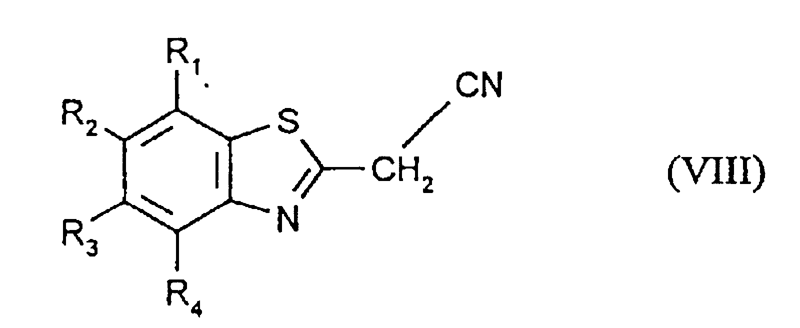 ou une amino-iso-indolénine de formule (IX) 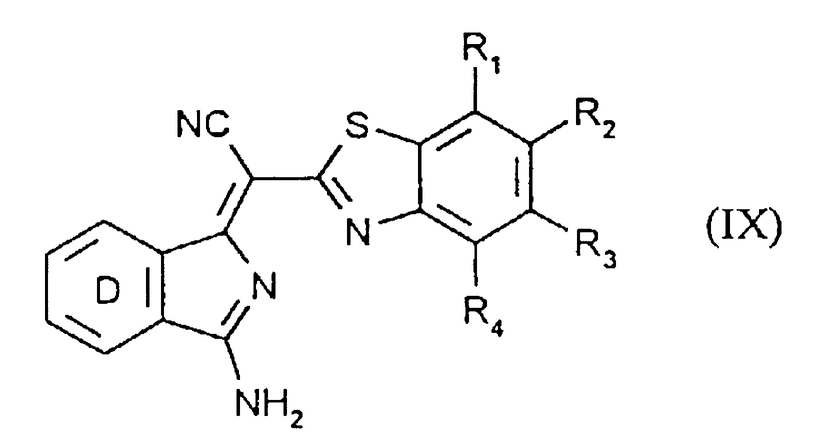 avec un ester d'acide cyanoacétique de formule (VI)
NC-CH₂-COOR₅ (VI),
formules dans lesquelles R₁ à R₅ et D ont les significations indiquées selon la revendication 3.

7. Procédé pour la préparation des colorants de formule (III) selon la revendication 4, **caractérisé en ce qu'**on condense des composés de formule (X) 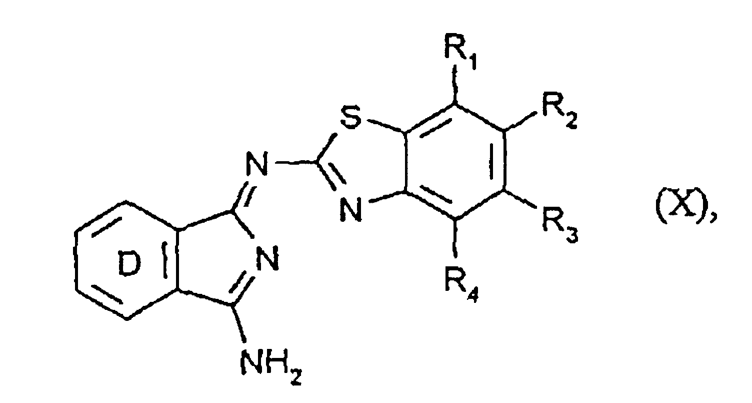 dans laquelle D et R₁ à R₄ ont les significations selon la revendication 4, avec des esters d'acide cyanoacétique de formule (VI)
NC-CH₂-COOR₅ (VI),
dans laquelle R₅ a la signification selon la revendication 4.

8. Procédé pour la préparation des composés de formule (IV) selon la revendication 5, **caractérisé en ce qu'**on fait réagir des amino-iso-indolénines de formule (IX) 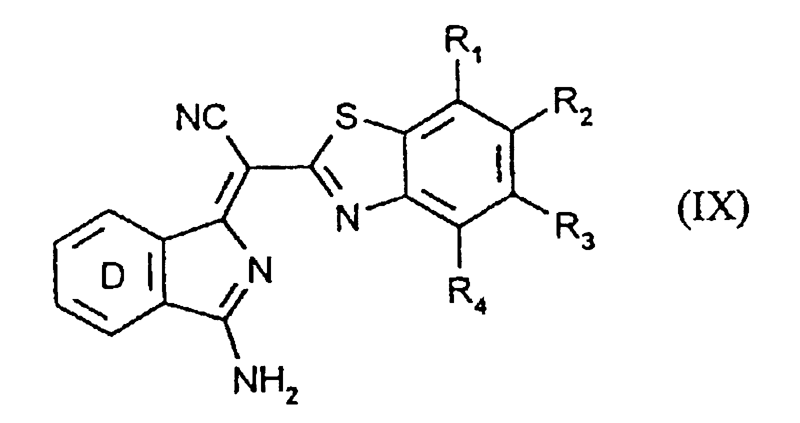 avec des amines de formule R₆-NH₂, D, R₁ à R₄ et R₆ ayant les significations selon la revendication 5.

9. Procédé pour la préparation des composés de formule (IV) selon la revendication 5, **caractérisé en ce qu'**on fait réagir des composés de formule (XIII) 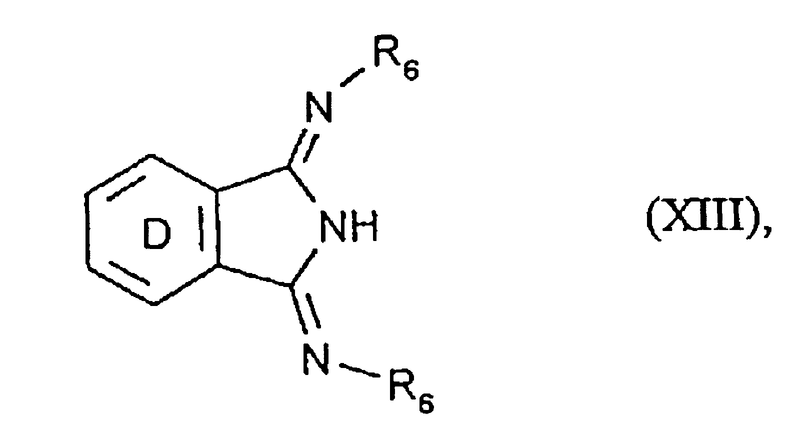 dans laquelle R₆ et D ont les significations données plus haut,
avec des composés de formule (VIII) 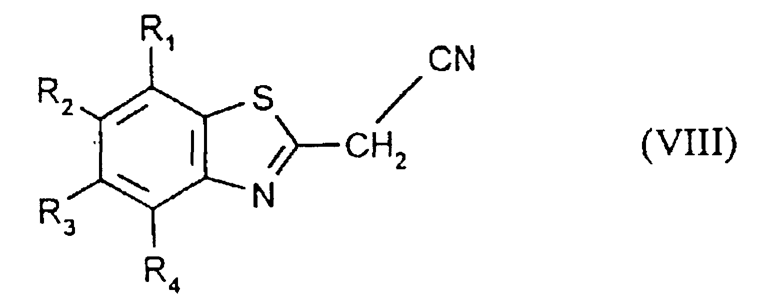 dans laquelle R₁ à R₄ ont les significations selon la revendication 5.

10. Utilisation des colorants de formule (I) selon la revendication 1, pour la teinture de matières entièrement synthétiques ou semi-synthétiques de masse moléculaire élevée.
